# EUROPEAN PATENT APPLICATION

(11) **EP 4 803 505 A1**
(43) Date of publication of application: **09.09.2026**
(21) Application number: 24885600.7
(22) Date of filing: 24.10.2024
(51) Int. Cl.: C07C 211/63, C07C 215/40, C07C 233/36

(54) **POLYACID SALT OR MIXTURE THEREOF**

(30) Priority: 31.10.2023 JP 2023187317
(71) Applicant: TOKYO OHKA KOGYO CO., LTD., Kawasaki-shi, Kanagawa 211 0012 (JP)
(72) Inventor: UNO, Kakishi, Kawasaki-shi, Kanagawa 211-0012 (JP); INARI, Takatoshi, Kawasaki-shi, Kanagawa 211-0012 (JP)
(74) Representative: Plasseraud IP
(86) International application number: PCT/JP2024/038005
(87) International publication number: WO 2025/094827

(57) **Abstract**

To provide a novel polyacid salt or a mixture thereof by introducing an organic quaternary ammonium cation, an organic quaternary ammonium dication, or a pyridinium cation, each having two or more ethylenically unsaturated double bonds, into a cation moiety of the polyacid salt.

## Description

### Technical Field

The present invention relates to a polyacid salt or a mixture thereof.

### Background Art

The polyacid is an anionic metal oxide cluster represented by the general formula [MₓO_{y}]ⁿ⁻ (wherein x, y, and n all denote natural numbers). The metal atom M constituting the polyacid is referred to as a polyatom and is, for example, Mo (hexavalent or pentavalent), W (hexavalent or pentavalent), V (pentavalent), Nb (pentavalent), Ta (pentavalent), or the like. The polyacid can be broadly divided into an isopolyacid composed of the polyatom M and an oxoacid, and a heteropolyacid ([X_{w}MₓO_{y}]ⁿ⁻ (wherein w, x, y, and n all denote natural numbers)) containing a different type of heteroatom X (for example, P⁵⁺, Si⁴⁺, Ge⁴⁺, B³⁺, S⁶⁺ or the like as a heteroatom X) in addition to the polyatom M and oxygen.

The physicochemical properties of a polyacid, such as redox ability, actinic radiation reactivity, and acid properties, can be changed depending on the combination and structure of the constituent elements, the type of counter cation, or the like.

For example, Patent Literature 1 describes that a polyacid salt containing a polyoxomolybdic acid and a compound with a pyridinium skeleton has a high photoluminescence quantum yield.

### Citation List

### Patent Literature

PTL 1: U.S. Patent No. 11192908

### Summary of Invention

### Technical Problem

An object of the present invention is to provide a novel polyacid salt or a mixture thereof.

### Solution to Problem

As a result of extensive studies to solve the above problems, the present inventors have completed the present invention by finding that a novel polyacid salt or a mixture thereof can be provided by introducing an organic quaternary ammonium cation, an organic quaternary ammonium dication, or a pyridinium cation, each having two or more ethylenically unsaturated double bonds, into a cation moiety of the polyacid salt.

More specifically, the present invention relates to the following inventions.
[1] A polyacid salt represented by the general formula (I) or a mixture thereof:

   (A^{m+})ₐ(Bⁿ⁺)_{b}(C^{(am+bn)-}) (I)

   In the formula (I),
   A^{m+} each independently denotes H⁺, a metal ion, or an ammonium ion,
   Bⁿ⁺ each independently denotes an organic quaternary ammonium cation, an organic quaternary ammonium dication, or a pyridinium cation, each having two or more ethylenically unsaturated double bonds,
   C^{(am+bn)-} denotes a polyacid anion, and
   m denotes an integer in the range of 1 to 5, n denotes an integer of 1 or 2, a denotes a real number, and b denotes a real number greater than 0.
[2] The polyacid salt or a mixture thereof according to [1], wherein the polyacid anion is an isopolyoxomolybdate anion, an isopolyoxotungstate anion, an isopolyoxoniobate anion, or an isopolyoxotantalate anion.
[3] The polyacid salt or a mixture thereof according to [1], wherein the polyacid anion is a heteropolyacid anion, and the heteropolyacid anion includes Mo, W, V, Nb, or Ta as a polyatom and includes P, Si, B, S, or Ge as a heteroatom.
[4] The polyacid salt or a mixture thereof according to [1], wherein the ethylenically unsaturated double bond is derived from a vinyl group, a (meth)acryloyl group, a (meth)acryloyloxy group, a (meth)acrylamide group, or a styryl group.
[5] The polyacid salt or a mixture thereof according to [1], wherein the organic quaternary ammonium cation having two or more ethylenically unsaturated double bonds is an organic quaternary ammonium cation represented by the general formula (II): 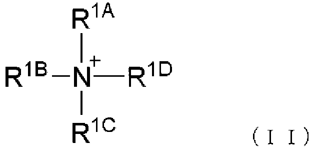 wherein
   R^{1A} to R^{1D} each independently denote a C₁₋₁₈ hydrocarbyl group, a 3- to 18-membered non-aromatic heterocyclic group, or a 5- to 18-membered aromatic heterocyclic group, the hydrocarbyl group and the heterocyclic groups each optionally having a substituent,
   a divalent carbon atom at any position of R^{1A} to R^{1D} except the terminal positions may be substituted with -O-, -C(=O)-, -C(=O)O-, -OCO-, -CONH-, -NHCO-, -NH(C=O)O-, -S-, or -SO₂- (provided that adjacent divalent carbon atoms are not substituted at the same time),
   a hydrogen atom in R^{1A} to R^{1D} may be substituted with (a) a fluorine atom, (b) a chlorine atom, (c) a bromine atom, (d) a haloalkyl group, (e) a hydroxy group, (f) a thiol group, (g) a nitro group, (h) a cyano group, (i) a carboxy group, (j) an amino group, (k) a sulfo group, (l) a vinyl group, (m) an allyl group, (n) a (meth)acryloyl group, (o) a (meth)acryloyloxy group, (p) a (meth)acrylamide group, (q) a styryl group, (r) an epoxy group, (s) a glycidyl group, (t) an amide group, (u) a hydroxy group or a carboxy group each having a protective group, or (v) a C₁₋₁₈ hydrocarbyl group, a C₁₋₁₈ hydrocarbyloxy group, a C₁₋₁₈ hydrocarbylcarbonyl group, a C₁₋₁₈ hydrocarbylcarbonyloxy group, a C₁₋₁₈ hydrocarbyloxycarbonyl group, a C₁₋₁₈ hydrocarbyloxycarbonyloxy group, a C₁₋₁₈ hydrocarbylamino group, a di-C₁₋₁₈ hydrocarbylamino group, a C₁₋₁₈ hydrocarbylaminocarbonyl group, a di-C₁₋₁₈ hydrocarbylaminocarbonyl group, a C₁₋₁₈ hydrocarbylcarbonylamino group, a C₁₋₁₈ hydrocarbylaminocarbonyloxy group, a di-C₁₋₁₈ hydrocarbylaminocarbonyloxy group, a C₁₋₁₈ hydrocarbylaminocarbonylamino group, a di-C₁₋₁₈ hydrocarbylaminocarbonylamino group, a C₁₋₁₈ hydrocarbyloxycarbonylamino group, or a C₁₋₁₈ hydrocarbylthio group, in which at least part of hydrogen atoms may be substituted with (a) to (u), and a divalent carbon atom at any position of these substituents except the terminal positions may be substituted with -O-, -C(=O)-, -C(=O)O-, -OCO-, -CONH-, - NHCO-, -NH(C=O)O-, -S-, or -SO₂- (provided that adjacent divalent carbon atoms are not substituted at the same time),
   at least one hydrogen atom in R^{1A} and R^{1B} is substituted with (l) a vinyl group, (m) an allyl group, (n) a (meth)acryloyl group, (o) a (meth)acryloyloxy group, (p) a (meth)acrylamide group, or (q) a styryl group, and
   any two of R^{1A} to R^{1D} may be directly bonded to each other by a single bond or may be bonded to each other via a divalent linking group -S-, -C(=O)-, -S(=O)-, -S(=O)₂-, - C(=O)O-, -C(=O)NH-, or a C₁₋₃ alkylene group to form a ring together with the nitrogen atom in the formula (II).
[6] The polyacid salt or a mixture thereof according to [1], wherein the organic quaternary ammonium cation having two or more ethylenically unsaturated double bonds is an organic quaternary ammonium cation represented by the general formula (III): 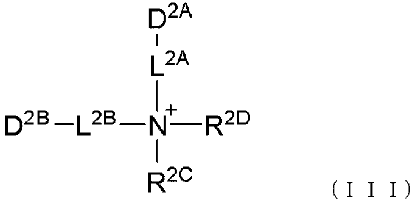 wherein
   L^{2A} and L^{2B} each independently denote an optionally substituted C₁₋₁₈ hydrocarbylene group,
   a divalent carbon atom at any position of L^{2A} and L^{2B} including the terminal positions may be substituted with -O-, -C(=O)-, -C(=O)O-, -OCO-, -CONH-, -NHCO-, -NH(C=O)O-, -S-, or -SO₂- (provided that adjacent divalent carbon atoms are not substituted at the same time),
   D^{2A} and D^{2B} each independently denote a vinyl group, a (meth)acryloyl group, a (meth)acryloyloxy group, a (meth)acrylamide group, or a styryl group,
   R^{2C} and R^{2D} each independently denote a C₁₋₁₈ hydrocarbyl group, a 3- to 18-membered non-aromatic heterocyclic group, or a 5- to 18-membered aromatic heterocyclic group, the hydrocarbyl group and the heterocyclic groups each optionally having a substituent,
   a divalent carbon atom at any position of R^{2C} and R^{2D} except the terminal positions may be substituted with -O-, -C(=O)-, -C(=O)O-, -OCO-, -CONH-, -NHCO-, -NH(C=O)O-, -S-, or -SO₂- (provided that adjacent divalent carbon atoms are not substituted at the same time),
   a hydrogen atom in L^{2A}, L^{2B}, R^{2C}, and R^{2D} may be substituted with (a) a fluorine atom, (b) a chlorine atom, (c) a bromine atom, (d) a C₁₋₃ haloalkyl group, (e) a hydroxy group, (f) a thiol group, (g) a nitro group, (h) a cyano group, (i) a carboxy group, (j) an amino group, (k) a sulfo group, (l) a vinyl group, (m) an allyl group, (n) a (meth)acryloyl group, (o) a (meth)acryloyloxy group, (p) a (meth)acrylamide group, (q) a styryl group, (r) an epoxy group, (s) a glycidyl group, (t) an amide group, (u) a hydroxy group or a carboxy group each having a protective group, or (v) a C₁₋₁₈ hydrocarbyl group, a C₁₋₁₈ hydrocarbyloxy group, a C₁₋₁₈ hydrocarbylcarbonyl group, a C₁₋₁₈ hydrocarbylcarbonyloxy group, a C₁₋₁₈ hydrocarbyloxycarbonyl group, a C₁₋₁₈ hydrocarbyloxycarbonyloxy group, a C₁₋₁₈ hydrocarbylamino group, a di-C₁₋₁₈ hydrocarbylamino group, a C₁₋₁₈ hydrocarbylaminocarbonyl group, a di-C₁₋₁₈ hydrocarbylaminocarbonyl group, a C₁₋₁₈ hydrocarbylcarbonylamino group, a C₁₋₁₈ hydrocarbylaminocarbonyloxy group, a di-C₁₋₁₈ hydrocarbylaminocarbonyloxy group, a C₁₋₁₈ hydrocarbylaminocarbonylamino group, a di-C₁₋₁₈ hydrocarbylaminocarbonylamino group, a C₁₋₁₈ hydrocarbyloxycarbonylamino group, or a C₁₋₁₈ hydrocarbylthio group, in which at least part of hydrogen atoms may be substituted with (a) to (u), and a divalent carbon atom at any position of these substituents except the terminal positions may be substituted with -O-, -C(=O)-, -C(=O)O-, -OCO-, -CONH-, - NHCO-, -NH(C=O)O-, -S-, or -SO₂- (provided that adjacent divalent carbon atoms are not substituted at the same time), and
   any two of L^{2A}, L^{2B}, R^{2C}, and R^{2D} may be directly bonded to each other by a single bond or may be bonded to each other via a divalent linking group -S-, -C(=O)-, -S(=O)-, - S(=O)₂-, -C(=O)O-, -C(=O)NH-, or a C₁₋₃ alkylene group to form a ring together with the nitrogen atom in the formula (III).

### Advantageous Effects of Invention

The present invention can provide a novel polyacid salt or a mixture thereof. It is possible to provide a novel functional building block having high actinic radiation (including visible light, ultraviolet radiation, X-rays, electron beams, α radiation, β radiation, γ radiation, and the like) reactivity by introducing an organic quaternary ammonium cation, an organic quaternary ammonium dication, or a pyridinium cation, each having two or more ethylenically unsaturated double bonds, into a cation moiety of a polyacid salt.

### Description of Embodiments

Preferred embodiments of the present invention will be described in detail below. However, the present invention is not limited to the following embodiments.

In the present description, the term "(meth)acryloyl group" is used to mean both an acryloyl group and a methacryloyl group. The same applies to other similar expressions.

The term "halogen atom", as used herein, refers to a fluorine atom, a chlorine atom, or a bromine atom.

In the present description, for example, the term "C₁₋₆" or the like means the number of carbon atoms of a group serving as a mother nucleus.

The term "C₁₋₁₈ hydrocarbylene group", as used herein, refers to a divalent hydrocarbon group formed by removing two hydrogen atoms from a hydrocarbon with 1 to 18 carbon atoms. The hydrocarbylene group may be linear, branched, or partially or fully cyclic. Examples of the hydrocarbylene group include an alkylene group and an arylene group.

A divalent carbon atom at any position of the "C₁₋₁₈ hydrocarbylene group" may be replaced by -O-, -S-, -C(=O)-, -COO-, -OCO-, -CONH-, -NHCO-, -NH(C=O)O-, -S-, -SO-, or -SO₂- (provided that adjacent divalent carbon atoms are not replaced at the same time).

The "C₁₋₁₈ hydrocarbylene group" is, for example, but not limited to, a "C₁₋₁₈ alkylene group", such as a methylene group, an ethylene group, a n-propylene group, an i-propylene group, a cyclopentadiyl group, a cyclohexanediyl group, an oxyethane-1,1-diyl group, an oxyethane-1,2-diyl group, an oxypropane-1,3-diyl group, an oxypropane-1,2-diyl group, a 2-methylpropane-1,3-diyl group, or an oxyethyleneoxyethane-1,1-diyl group; a "C₆₋₁₈ arylene group", such as a 1,4-phenylene group, a 1,3-phenylene group, a 1,2-phenylene group, a 1,4-naphthylene group, a 1,5-naphthylene group, a 1,8-naphthylene group, a 4,4'-biphenylene group, an anthracenediyl group, a phenanthrenediyl group, a naphthacenediyl group, a pyrenediyl group, a perylenediyl group, or a chrysenediyl group; or the like.

The term "C₁₋₁₈ alkyl group", as used herein, refers to a linear or branched alkyl group with 1 to 18 carbon atoms.

Furthermore, a divalent carbon atom at any position excluding the terminals contained in the "C₁₋₁₈ alkyl group" may be replaced by -O-, -C(=O)-, -C(=O)O-, -OCO-, - CONH-, -NHCO-, -NH(C=O)O-, -S-, or -SO₂-. However, adjacent divalent carbon atoms are not replaced at the same time.

The "C₁₋₁₈ alkyl group" is, for example, but not limited to, a methyl group, an ethyl group, a n-propyl group, an i-propyl group, a n-butyl group, an i-butyl group, a sec-butyl group, a t-butyl group, a n-pentyl group, an i-pentyl group, a sec-pentyl group, a t-pentyl group, a neopentyl group, a 1-methylbutyl group, a 2-methylbutyl group, a 1,1-dimethylpropyl group, a 1,2-dimethylpropyl group, a n-hexyl group, an i-hexyl group, a 1-methylpentyl group, a 2-methylpentyl group, a 3-methylpentyl group, a 1,1-dimethylbutyl group, a 1,2-dimethylbutyl group, a 2,2-dimethylbutyl group, a 1,3-dimethylbutyl group, a 2,3-dimethylbutyl group, a 3,3-dimethylbutyl group, a 1-ethylbutyl group, a 2-ethylbutyl group, a 1,1,2-trimethylpropyl group, a 1,2,2-trimethylpropyl group, a 1-ethyl-1-methylpropyl group, a 1-ethyl-2-methylpropyl group, a n-heptyl group, a n-octyl group, a n-nonyl group, a n-decyl group, a n-undecyl group, a n-dodecyl group, or the like.

The "C₁₋₁₈ alkyl group" in which a divalent carbon atom at any position excluding the terminals thereof is replaced by -O-, -C(=O)-, -C(=O)O-, -OCO-, -CONH-, -NHCO-, - NH(C=O)O-, -S-, or -SO₂- is, for example, but not limited to, a 2-methoxyethoxymethyl group, an ethoxycarbonylmethyl group, or the like.

The term "C₁₋₁₈ haloalkyl group", as used herein, refers to a group in which one or more hydrogen atoms of the "C₁₋₁₈ alkyl group" are substituted by a halogen atom.

The "C₁₋₁₈ haloalkyl group" is, for example, but not limited to, a dichloromethyl group, a trifluoromethyl group, a 2,2-difluoroethyl group, a 2,2,2-trifluoroethyl group, a pentafluoroethyl group, a 3,3,3-trifluoropropyl group, or the like.

The term "C₂₋₁₈ alkenyl group", as used herein, refers to an alkenyl group with 2 or more carbon atoms having one or more double bonds in the "C₁₋₁₈ alkyl group" and includes an alkadienyl group, an alkatrienyl group, and the like.

The "C₂₋₁₈ alkenyl group" is, for example, but not limited to, a vinyl group (ethenyl group), an allyl group (2-propenyl group), a 1-propenyl group, an isopropenyl group (1-methyl vinyl group), a 1-butenyl group, a 2-butenyl group, a 3-butenyl group, a pentenyl group, a hexenyl group, a heptenyl group, an octenyl group, a nonenyl group, a decenyl group, an undecenyl group, a dodecenyl group, or the like.

The term "C₂₋₁₈ alkynyl group", as used herein, refers to an alkynyl group with 2 or more carbon atoms having one or more triple bonds in the "C₁₋₁₈ alkyl group".

The "C₂₋₁₈ alkynyl group" is, for example, but not limited to, an ethynyl group, a 1-propynyl group, a 2-propynyl group, a pentynyl group, a hexynyl group, a heptynyl group, an octynyl group, a nonynyl group, a decynyl group, an undecynyl group, a dodecynyl group, or the like.

The term "C₃₋₁₈ alicyclic group", as used herein, refers to a hydrocarbon group with 3 to 18 carbon atoms and with a monocyclic or polycyclic structure in whole or in part. The alicyclic group includes a cycloalkyl group, a cycloalkenyl group, a cycloalkynyl group, a monocycloalkyl group, a polycycloalkyl group, and the like.

A divalent carbon atom at any position excluding the terminals contained in the "C₃₋₁₈ alicyclic group" may be replaced by -O-, -C(=O)-, -C(=O)O-, -OCO-, -CONH-, -NHCO-, -NH(C=O)O-, -S-, or -SO₂- (provided that adjacent divalent carbon atoms are not replaced at the same time).

The "C₃₋₁₈ cycloalkyl group" is, for example, but not limited to, a cyclopropyl group, a cyclobutyl group, a cyclopentyl group, a 1-i-propylcyclopentane-1-yl group, a cyclohexyl group, a t-butylcyclohexyl group, a tricyclodecanyl group, a cycloheptyl group, a cyclooctyl group, a cyclodecyl group, a 2-methyladamantane-2-yl group, a 2-i-propyladamantane-2-yl group, a bornyl group, a norbonyl group, a fenchyl group, a pinanyl group, an adamantyl group, a tricyclodecyl group, a tetracyclododecyl group, a cyclopropylmethyl group, a cyclobutylmethyl group, a cyclopentylmethyl group, a cyclohexylmethyl group, a bornylmethyl group, a norbornylmethyl group, an adamantylmethyl group, a 1-methylcyclopentyloxycarbonylmethyl group, a cyclopentenyl group, a cyclohexenyl group, a cycloheptenyl group, or the like.

The term "3- to 18-membered non-aromatic heterocyclic group", as used herein, refers to a 3- to 18-membered non-aromatic heterocyclic group containing one or more heteroatoms selected from the group consisting of a nitrogen atom, an oxygen atom, and a sulfur atom, may be monocyclic, polycyclic, or condensed, and may be saturated or partially unsaturated.

The "3- to 18-membered non-aromatic heterocyclic group" is, for example, but not limited to, an aziridinyl group, an azetidil group, a pyrrolidinyl group, a pyrrolyl group, a piperidinyl group, a piperazinyl group, a morpholinyl group, a thiomorpholinyl group, a tetrahydrofuryl group, a tetrahydropyranyl group, an oxetanyl group, a tetrahydrofuryl group, a tetrahydropyranyl group, an imidazolinyl group, an oxazolinyl group, a 2,5-diazabicyclo[2.2.1]heptyl group, a 2,5-diazabicyclo[2.2.2]octyl group, a 3,8-diazabicyclo[3.2.1]octyl group, a 1,4-diazabicyclo[4.3.0]nonyl group, a 1-azaadamantyl group, a 2-azaadamantyl group, or the like.

The term "C₂₋₁₈ aryl group", as used herein, refers to an aromatic hydrocarbon ring group with 6 to 18 carbon atoms or an aromatic heterocyclic group with 2 to 10 carbon atoms; in the case of an aromatic heterocyclic group, 2 to 10 carbon atoms and one or more heteroatoms selected from the group consisting of a nitrogen atom, an oxygen atom, and a sulfur atom form a ring, and the ring may be monocyclic, polycyclic, or a fused ring.

The "C₂₋₁₈ aryl group" is, for example, but not limited to, a phenyl group, a 1-naphthyl group, a 2-naphthyl group, an azulenyl group, a pentalenyl group, a heptalenyl group, an indacenyl group, an acenaphthyl group, a phenanthrenyl group, an anthracenyl group, or the like.

The term "C₇₋₁₈ aralkyl group", as used herein, refers to a group in which a substitutable portion of a "C₁₋₁₂ alkyl group" is substituted by the "C₂₋₁₂ aryl group".

The "C₇₋₁₈ aralkyl group" is, for example, but not limited to, a benzyl group, a phenethyl group, a 3-phenylpropyl group, a 4-phenylbutyl group, a 1-naphthylmethyl group, or a 2-naphthylmethyl group, or the like.

The term "C₁₋₁₈ hydrocarbyl group", as used herein, refers to a monovalent group formed by removing one hydrogen atom from a hydrocarbon with 1 to 18 carbon atoms. Examples of the hydrocarbyl group include an alkyl group, an alkenyl group, an alkynyl group, an alicyclic group, an aryl group, an aralkyl group, and the like.

A divalent carbon atom at any position excluding the terminals contained in the "C₁₋₁₈ hydrocarbyl group" may be replaced by -O-, -C(=O)-, -C(=O)O-, -OCO-, -CONH-, - NHCO-, -NH(C=O)O-, -S-, or -SO₂- (provided that adjacent divalent carbon atoms are not replaced at the same time). The same applies to the "C₁₋₁₈ hydrocarbyl group" and the like used in the following description of the definition of the "C₁₋₁₈ hydrocarbyloxy group" and the like.

The "C₁₋₁₈ hydrocarbyl group" is, for example, but not limited to, a "C₁₋₁₈ alkyl group", a "C₂₋₁₈ alkenyl group", a "C₂₋₁₈ alkynyl group", a "C₃₋₁₈ alicyclic group", a "C₂₋₁₈ aryl group", a "C₇₋₁₈ aralkyl group", or the like.

The term "C₁₋₁₈ hydrocarbyloxy group", as used herein, refers to a group in which an oxygen atom (-O-) is bonded to the "C₁₋₁₈ hydrocarbyl group".

The "C₁₋₁₈ hydrocarbyloxy group" is, for example, but not limited to, a "C₁₋₁₈ alkoxy group", such as a methoxy group, an ethoxy group, a n-propoxy group, an i-propoxy group, a n-butoxy group, an i-butoxy group, a sec-butoxy group, a t-butoxy group, a n-pentoxy group, an i-pentoxy group, a sec-pentoxy group, a n-hexoxy group, an i-hexoxy group, a 1,1-dimethylpropyloxy group, a 1,2-dimethylpropyloxy group, a 2,2-dimethylpropyloxy group, a 1-methyl-2-ethylpropyloxy group, a 1-ethyl-2-methylpropyloxy group, a 1,1,2-trimethylpropyloxy group, a 1,2,2-trimethylpropyloxy group, a 1,1-dimethylbutyloxy group, a 1,2-dimethylbutyloxy group, a 2,2-dimethylbutyloxy group, a 2,3-dimethylbutyloxy group, a 1,3-dimethylbutyloxy group, a 2-ethylbutyloxy group, a 2-methylpentyloxy group, or a 3-methylpentyloxy group; a "C₃₋₁₈ alicyclic oxy group", such as a cyclopropyloxy group, a cyclobutyloxy group, a cyclopentyloxy group, a cyclohexyloxy group, a cycloheptyloxy group, a cyclooctyloxy group, a 1-methylcyclopentyloxycarbonylmethoxy group, a 1-ethylcyclohexyloxycarbonylmethoxy group, or a 1-methyladamantyloxycarbonylmethoxy group; a "C₆₋₁₈ aryloxy group", such as a phenyloxy group, a 1-naphthyloxy group, a 2-naphthyloxy group, an azulenyloxy group, a pentalenyloxy group, a heptalenyloxy group, an indacenyloxy group, an acenaphthyloxy group, a phenanthrenyloxy group, or an anthracenyloxy group; or the like.

In the "C₁₋₁₈ hydrocarbyloxy group", a divalent carbon atom at any position excluding the terminals in the "C₁₋₁₈ hydrocarbyl group" is preferably replaced by -O-, - C(=O)-, and/or -C(=O)O-, the "C₁₋₁₈ hydrocarbyloxy group" is more preferably a "C₁₋₁₈ hydrocarbyloxycarbonylalkyloxy group", and from the perspective of solubility, a carbon atom bonded to the oxygen atom of the hydrocarbyloxy is still more preferably a tertiary carbon. Specific examples of the hydrocarbyloxy include optionally substituted ethylcyclopentyloxy, methyladamantyloxy, ethyladamantyloxy, t-butyloxy, and the like.

The term "C₁₋₁₈ hydrocarbylcarbonyl group", as used herein, refers to a group in which a carbonyl group (-C(=O)-) is bonded to the "C₁₋₁₈ hydrocarbyl group".

The "C₁₋₁₈ hydrocarbylcarbonyl group" is, for example, but not limited to, a "C₁₋₁₈ alkyl carbonyl group", such as an acetyl group, a propionyl group, an isopropionyl group, a butyryl group, an isobutyryl group, a valeryl group, an isovaleryl group, a pentanoyl group, a 3-methylbutanoyl group, a pivaloyl group, a hexanoyl group, or a heptanoyl group; a "C₃₋₁₈ alicyclic carbonyl group", such as a cyclopropyl carbonyl group, a cyclobutyl carbonyl group, a cyclopentyl carbonyl group, a 2-methylcyclopentyl carbonyl group, a 3-methylcyclopentyl carbonyl group, a cyclohexyl carbonyl group, a 2-methylcyclohexyl carbonyl group, a 3-methylcyclohexyl carbonyl group, a 4-methylcyclohexyl carbonyl group, or an adamantyl carbonyl group; a "C₆₋₁₈ aryl carbonyl group", such as a benzoyl group, a 1-naphthoyl group, or a 2-naphthoyl group; or the like.

The term "C₁₋₁₈ hydrocarbylcarbonyloxy group", as used herein, refers to a group in which an oxygen atom (-O-) is bonded to the "C₁₋₁₈ hydrocarbylcarbonyl group".

The "C₁₋₁₈ hydrocarbylcarbonyloxy group" is, for example, but not limited to, a "C₁₋₁₈ alkyl carbonyloxy group", such as a methyl carbonyloxy group, an ethyl carbonyloxy group, a n-propyl carbonyloxy group, an isopropyl carbonyloxy group, a n-butyl carbonyloxy group, an isobutyl carbonyloxy group, a t-butyl carbonyloxy group, a n-pentyl carbonyloxy group, an isopentyl carbonyloxy group, or a hexyl carbonyloxy group; a "C₃₋₁₈ alicyclic carbonyloxy group", such as a cyclopropyl carbonyloxy group, a cyclobutyl carbonyloxy group, a cyclopentyl carbonyloxy group, or a cyclohexyl carbonyloxy group; a "C₆₋₁₈ aryl carbonyloxy group", such as a phenyl carbonyloxy group, a naphthyl carbonyloxy group, an acenaphthyl carbonyloxy group, a phenanthrenyl carbonyloxy group, or an anthracenyl carbonyloxy group; or the like.

The term "C₁₋₁₈ hydrocarbyloxycarbonyl group", as used herein, refers to a group in which a carbonyl group (-C(=O)-) is bonded to a "C₁₋₁₈ hydrocarbyloxy group".

The "C₁₋₁₈ hydrocarbyloxycarbonyl group" is, for example, but not limited to, a "C₁₋₁₈ alkoxycarbonyl group", such as a methoxycarbonyl group, an ethoxycarbonyl group, a n-propoxycarbonyl group, an i-propoxycarbonyl group, a n-butoxycarbonyl group, an i-butoxycarbonyl group, a sec-butoxycarbonyl group, a t-butoxycarbonyl group, a n-pentoxycarbonyl group, or a neopentyloxycarbonyl group; a "C₃₋₁₈ alicyclic oxycarbonyl group", such as a cyclopropyloxycarbonyl group, a cyclobutyloxycarbonyl group, a cyclopentyloxycarbonyl group, a cyclohexyloxycarbonyl group, a 2-methylcyclopentyloxycarbonyl group, a 3-methylcyclopentyloxycarbonyl group, a 2-methylcyclohexyloxycarbonyl group, a 3-methylcyclohexyloxycarbonyl group, or a 4-methylcyclohexyloxycarbonyl group; a "C₆₋₁₈ aryloxycarbonyl group", such as a phenoxycarbonyl group, a naphthoxycarbonyl group, an acenaphthyloxycarbonyl group, a phenanthrenyloxycarbonyl group, or an anthracenyloxycarbonyl group; or the like.

The term "C₁₋₁₈ hydrocarbyloxycarbonyloxy group", as used herein, refers to a group in which an oxygen atom (-O-) is bonded to the "C₁₋₁₈ hydrocarbyloxycarbonyl group".

The "C₁₋₁₈ hydrocarbyloxycarbonyloxy group" is, for example, but not limited to, a "C₁₋₁₈ alkoxycarbonyloxy group", such as a methoxycarbonyloxy group, an ethoxycarbonyloxy group, a n-propyloxycarbonyloxy group, an i-propyloxycarbonyloxy group, a n-butoxycarbonyloxy group, an i-butoxycarbonyloxy group, a sec-butoxycarbonyloxy group, a t-butoxycarbonyloxy group, a n-pentyloxycarbonyloxy group, an i-pentyloxycarbonyloxy group, or a n-hexyloxycarbonyloxy group; a "C₃₋₁₈ alicyclic oxycarbonyloxy group", such as a cyclopropyloxycarbonyloxy group, a cyclobutyloxycarbonyloxy group, a cyclopentyloxycarbonyloxy group, or a cyclohexyloxycarbonyloxy group; a "C₆₋₁₈ aryloxycarbonyloxy group", such as a phenoxycarbonyloxy group, a naphthoxycarbonyloxy group, an acenaphthyloxycarbonyloxy group, a phenanthrenyloxycarbonyloxy group, or an anthracenyloxycarbonyloxy group; or the like.

The term "C₁₋₁₈ hydrocarbylamino group", as used herein, refers to a group in which one "C₁₋₁₈ hydrocarbyl group" is bonded to an amino group.

The "C₁₋₁₈ hydrocarbylamino group" is, for example, but not limited to, a "C₁₋₁₈ alkylamino group", such as a methylamino group, an ethylamino group, a n-propylamino group, an i-propylamino group, a n-butylamino group, an i-butylamino group, a sec-butylamino group, a t-butylamino group, a n-pentylamino group, an i-pentylamino group, a neopentylamino group, or a n-hexylamino group; a "C₃₋₁₈ alicyclic amino group", such as a cyclopropylamino group, a cyclobutylamino group, a cyclopentylamino group, a 2-methylcyclopentylamino group, a 3-methylcyclopentylamino group, a cyclohexylamino group, a 2-methylcyclohexylamino group, a 3-methylcyclohexylamino group, or a 4-methylcyclohexylamino group; a "C₆₋₁₈ arylamino group", such as a phenylamino group, a 1-naphthylamino group, or a 2-naphthylamino group; or the like.

The term "di-C₁₋₁₈ hydrocarbylamino group", as used herein, refers to a group in which two identical or different "C₁₋₁₈ hydrocarbyl groups" are bonded to an amino group.

The "di-C₁₋₁₈ hydrocarbylamino group" is, for example, but not limited to, a "di-C₁₋₁₈ alkylamino group", such as a dimethylamino group, a diethylamino group, a di-n-propylamino group, a diisopropylamino group, a di-n-butylamino group, a diisobutylamino group, a di-t-butylamino group, a di-n-pentylamino group, a di-n-hexylamino group, an N-ethyl-N-methylamino group, an N-methyl-N-n-propylamino group, an N-isopropyl-N-methylamino group, an N-n-butyl-N-methylamino group, an N-isobutyl-N-methylamino group, an N-t-butyl-N-methylamino group, an N-methyl-N-n-pentylamino group, an N-n-hexyl-N-methylamino group, an N-ethyl-N-n-propylamino group, an N-ethyl-N-isopropylamino group, an N-n-butyl-N-ethylamino group, an N-ethyl-N-isobutylamino group, an N-t-butyl-N-ethylamino group, an N-ethyl-N-n-pentylamino group, or an N-ethyl-N-n-hexylamino group; a "di-C₃₋₁₈ alicyclic amino group", such as a dicyclopropylamino group, a dicyclobutylamino group, a dicyclopentylamino group, or a dicyclohexylamino group; a "di-C₆₋₁₈ arylamino group", such as a diphenylamino group or a phenylnaphthylamino group; an "N-C₁₋₁₈ alkyl-N-C₃₋₁₈ cycloalkylamino group", such as an N-methylcyclopentaneamino group or an N-methylcyclohexylamino group; an "N-C₁₋₁₈ alkyl-N-C₆₋₁₈ arylamino group", such as an N-methyl-2-phenylethylamino group or an N-ethyl-N-(4-methylphenyl)amino group; or the like.

The term "C₁₋₁₈ hydrocarbylaminocarbonyl group", as used herein, refers to a group in which a carbonyl group (-C(=O)-) is bonded to the "C₁₋₁₈ hydrocarbylamino group".

The "C₁₋₁₈ hydrocarbylaminocarbonyl group" is, for example, but not limited to, a "C₁₋₁₈ alkylaminocarbonyl group", such as a methylaminocarbonyl group, an ethylaminocarbonyl group, a n-propylaminocarbonyl group, an i-propylaminocarbonyl group, a n-butylaminocarbonyl group, a sec-butylaminocarbonyl group, a t-butylaminocarbonyl group, a n-pentylaminocarbonyl group, a 2-pentylaminocarbonyl group, a neopentylaminocarbonyl group, a 4-methyl-2-pentylaminocarbonyl group, a n-hexylaminocarbonyl group, or a 3-methyl-n-pentylaminocarbonyl group; a "C₃₋₁₈ alicyclic aminocarbonyl group", such as a cyclopropylaminocarbonyl group, a cyclobutylaminocarbonyl group, a cyclopentylaminocarbonyl group, a cyclohexylaminocarbonyl group, a 2-methylcyclopentylaminocarbonyl group, a 3-methylcyclopentylaminocarbonyl group, a 2-methylcyclohexylaminocarbonyl group, a 3-methylcyclohexylaminocarbonyl group, or a 4-methylcyclohexylaminocarbonyl group; or a "C₆₋₁₈ arylaminocarbonyl group", such as a phenylaminocarbonyl group, a 1-naphthylaminocarbonyl group, or a 2-naphthylaminocarbonyl group.

The term "di-C₁₋₁₈ hydrocarbylaminocarbonyl group", as used herein, refers to a group in which a carbonyl group (-C(=O)-) is bonded to a "di-C₁₋₁₈ hydrocarbylamino group".

The "di-C₁₋₁₈ hydrocarbylaminocarbonyl group" is, for example, but not limited to, a "di-C₁₋₁₈ alkylamino group", such as a dimethylaminocarbonyl group, a diethylaminocarbonyl group, a di-n-propylaminocarbonyl group, a diisopropylaminocarbonyl group, a di-n-butylaminocarbonyl group, a diisobutylaminocarbonyl group, a di-t-butylaminocarbonyl group, a di-n-pentylaminocarbonyl group, a di-n-hexylaminocarbonyl group, an N-ethyl-N-methylaminocarbonyl group, an N-methyl-N-n-propylaminocarbonyl group, an N-isopropyl-N-methylaminocarbonyl group, an N-n-butyl-N-methylaminocarbonyl group, an N-isobutyl-N-methylaminocarbonyl group, an N-t-butyl-N-methylaminocarbonyl group, an N-methyl-N-n-pentylaminocarbonyl group, an N-n-hexyl-N-methylaminocarbonyl group, an N-ethyl-N-n-propylaminocarbonyl group, an N-ethyl-N-isopropylaminocarbonyl group, an N-n-butyl-N-ethylaminocarbonyl group, an N-ethyl-N-isobutylaminocarbonyl group, an N-t-butyl-N-ethylaminocarbonyl group, an N-ethyl-N-n-pentylaminocarbonyl group, or an N-ethyl-N-n-hexylaminocarbonyl group; a "di-C₃₋₁₈ alicyclic aminocarbonyl group", such as a dicyclopropylaminocarbonyl group, a dicyclobutylaminocarbonyl group, a dicyclopentylaminocarbonyl group, or a dicyclohexylaminocarbonyl group; a "di-C₆₋₁₈ arylaminocarbonyl group", such as a diphenylaminocarbonyl group or a phenylnaphthylaminocarbonyl group; or the like.

The term "C₁₋₁₈ hydrocarbylcarbonylamino group", as used herein, refers to a group in which an amino group is bonded to the "C₁₋₁₈ hydrocarbylcarbonyl group".

The "C₁₋₁₈ hydrocarbylcarbonylamino group" is, for example, but not limited to, a "C₁₋₁₈ alkyl carbonylamino group", such as a methyl carbonylamino group, an ethyl carbonylamino group, a n-propyl carbonylamino group, an i-propyl carbonylamino group, a n-butyl carbonylamino group, an i-butyl carbonylamino group, a sec-butyl carbonylamino group, a t-butyl carbonylamino group, a n-pentyl carbonylamino group, an i-pentyl carbonylamino group, or a n-hexyl carbonylamino group; a "C₃₋₁₈ alicyclic carbonylamino group", such as a cyclopropyl carbonylamino group, a cyclobutyl carbonylamino group, a cyclopentyl carbonylamino group, or a cyclohexyl carbonylamino group; a "C₆₋₁₈ aryl carbonylamino group", such as a phenyl carbonylamino group, a naphthyl carbonylamino group, an acenaphthyl carbonylamino group, a phenanthrenyl carbonylamino group, or an anthracenyl carbonyl amino group; or the like.

The term "C₁₋₁₈ hydrocarbylaminocarbonyloxy group", as used herein, refers to a group in which an oxygen atom (-O-) is bonded to the "C₁₋₁₈ hydrocarbylaminocarbonyl group".

The "C₁₋₁₈ hydrocarbylaminocarbonyloxy group" is, for example, but not limited to, a "C₁₋₁₈ alkylaminocarbonyloxy group", such as a methylaminocarbonyloxy group, an ethylaminocarbonyloxy group, or a n-propylaminocarbonyloxy group; a "C₃₋₁₈ alicyclic aminocarbonyloxy group", such as a cyclopropylaminocarbonyloxy group or a cyclohexylaminocarbonyloxy group; a "C₆₋₁₈ arylaminocarbonyloxy group", such as a phenylaminocarbonyloxy group or a 1-naphthylaminocarbonyloxy group; or the like.

The term "di-C₁₋₁₈ hydrocarbylaminocarbonyloxy group", as used herein, refers to a group in which an oxygen atom (-O-) is bonded to the "di-C₁₋₁₈ hydrocarbylaminocarbonyl group".

The "di-C₁₋₁₈ hydrocarbylaminocarbonyloxy group" is, for example, but not limited to, a "di-C₁₋₁₈ alkylaminocarbonyloxy group", such as a dimethylaminocarbonyloxy group, a diethylaminocarbonyloxy group, or a di-n-propylaminocarbonyloxy group; or the like.

The term "C₁₋₁₈ hydrocarbylaminocarbonylamino group", as used herein, refers to a group in which the "C₁₋₁₈ hydrocarbylaminocarbonyl group" is bonded to an amino group.

The "C₁₋₁₈ hydrocarbylaminocarbonylamino group" is, for example, but not limited to, a "C₁₋₁₈ alkylaminocarbonylamino group", such as a methylaminocarbonylamino group, an ethylaminocarbonyloxy group, or a n-propylaminocarbonylamino group; a "C₃₋₁₈ alicyclic aminocarbonylamino group", such as a cyclopropylaminocarbonylamino group or a cyclohexylaminocarbonylamino group; a "C₆₋₁₈ arylaminocarbonylamino group", such as a phenylaminocarbonylamino group or a 1-naphthylaminocarbonylamino group; or the like.

The term "di-C₁₋₁₈ hydrocarbylaminocarbonylamino group", as used herein, refers to a group in which a "di-C₁₋₁₈ hydrocarbylaminocarbonyl group" is bonded to an amino group.

The "di-C₁₋₁₈ hydrocarbylaminocarbonylamino group" is, for example, but not limited to, a "di-C₁₋₁₈ alkylaminocarbonylamino group", such as a dimethylaminocarbonylamino group, a diethylaminocarbonylamino group, or a di-n-propylaminocarbonylamino group; or the like.

The term "C₁₋₁₈ hydrocarbyloxycarbonylamino group", as used herein, refers to a group in which the "C₁₋₁₈ hydrocarbyloxycarbonyl group" is bonded to an amino group.

The "C₁₋₁₈ hydrocarbyloxycarbonylamino group" is, for example, but not limited to, a "C₁₋₁₈ alkoxycarbonylamino group", such as a methoxycarbonylamino group, an ethoxycarbonylamino group, a n-propoxycarbonylamino group, an i-propoxycarbonylamino group, a n-butoxycarbonylamino group, or a t-butoxycarbonylamino group; a "C₃₋₁₈ alicyclic oxycarbonylamino group", such as a cyclopropyloxycarbonylamino group or a cyclohexyloxycarbonylamino group; a "C₆₋₁₈ aryloxycarbonylamino group", such as a phenyloxycarbonylamino group or a 1-naphthyloxycarbonylamino group; or the like.

The term "C₁₋₁₈ hydrocarbylthio group", as used herein, refers to a group in which a sulfur atom (-S-) is bonded to the "C₁₋₁₈ hydrocarbyl group".

The "C₁₋₁₈ hydrocarbylthio group" is, for example, but not limited to, a "C₁₋₁₈ alkylthio group", such as a methylthio group, an ethylthio group, a n-propylthio group, an i-propylthio group, a n-butylthio group, an i-butylthio group, a t-butylthio group, a n-pentylthio group, or a n-hexylthio group; a "C₃₋₁₈ alicyclic thio group", such as a cyclopropylthio group, a cyclobutylthio group, a cyclopentylthio group, a cyclohexylthio group, a 2-methylcyclopentylthio group, a 3-methylcyclopentylthio group, a 2-methylcyclohexylthio group, a 3-methylcyclohexylthio group, or a 4-methylcyclohexylthio group; a "C₆₋₁₈ arylthio group", such as a phenylthio group, a 1-naphthylthio group, a 2-naphthylthio group, an acenaphthylthio group, a phenanthrenylthio group, or an anthracenylthio group; or the like.

The term "C₁₋₁₈ hydrocarbylsulfinyl group", as used herein, refers to a group in which a sulfinyl group (-S(=O)-) is bonded to the "C₁₋₁₈ hydrocarbyl group".

The "C₁₋₁₈ hydrocarbylsulfinyl group" is, for example, but not limited to, a "C₁₋₁₈ alkylsulfinyl group", such as a methylsulfinyl group, an ethylsulfinyl group, a n-propylsulfinyl group, an i-propylsulfinyl group, a n-butylsulfinyl group, a t-butylsulfinyl group, a pentylsulfinyl group, or a hexylsulfinyl group; a "C₃₋₁₈ alicyclic sulfinyl group", such as a cyclopropylsulfinyl group, a cyclobutylsulfinyl group, a cyclopentylsulfinyl group, a cyclohexylsulfinyl group, a 2-methylcyclopentylsulfinyl group, a 3-methylcyclopentylsulfinyl group, a 2-methylcyclohexylsulfinyl group, a 3-methylcyclohexylsulfinyl group, or a 4-methylcyclohexylsulfinyl group; a "C₆₋₁₈ arylsulfinyl group", such as a phenylsulfinyl group, a naphthylsulfinyl group, an acenaphthylsulfinyl group, a phenanthrenylsulfinyl group, or an anthracenylsulfinyl group; or the like.

The term "C₁₋₁₈ hydrocarbylsulfonyl group", as used herein, refers to a group in which a sulfonyl group (-SO₂-) is bonded to the "C₁₋₁₈ hydrocarbyl group".

The "C₁₋₁₈ hydrocarbylsulfonyl group" is, for example, but not limited to, a "C₁₋₁₈ alkylsulfonyl group", such as a methylsulfonyl group, an ethylsulfonyl group, a n-propylsulfonyl group, an i-propylsulfonyl group, a n-butylsulfonyl group, a t-butylsulfonyl group, or a pentylsulfonyl group; a "C₃₋₁₈ alicyclic sulfonyl group", such as a cyclopropylsulfonyl group, a cyclobutylsulfonyl group, a cyclopentylsulfonyl group, a cyclohexylsulfonyl group, a 2-methylcyclopentylsulfonyl group, a 3-methylcyclopentylsulfonyl group, a 2-methylcyclohexylsulfonyl group, a 3-methylcyclohexylsulfonyl group, or a 4-methylcyclohexyl group; a "C₆₋₁₈ arylsulfonyl group", such as a phenylsulfonyl group, a naphthylsulfonyl group, an acenaphthylsulfonyl group, a phenanthrenylsulfonyl group, or an anthracenylsulfonyl group; or the like.

The term "acid-dissociable group", as used herein, refers to a group that substitutes for a hydrogen atom of a carboxy group and a hydroxy group (including a phenolic hydroxy group and the like) and that is dissociated by the action of an acid.

The acid-dissociable group is, for example, an acid-dissociable group G for a hydroxy group or a carboxy group represented by the following general formula (G-1) or (G-2).

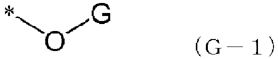

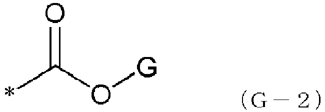

The acid-dissociable group G is not particularly limited, provided that it can be dissociated by the action of an acid, and a known and commonly used group can be used. The acid-dissociable group G is, for example, a "tertiary-carbon-type acid-dissociable group G_{A}" represented by the following general formula (g-1), or the like.

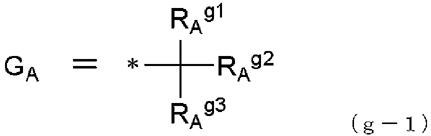

### <Tertiary-carbon-type Acid-Dissociable Group G_{A}>

As the acid-dissociable group G, an acid-dissociable group in which a carbon that is directly bonded to a polar group and to which R_{A}^{g1} to R_{A}^{g3} are bonded is a tertiary carbon atom, such as a "tertiary-carbon-type acid-dissociable group G_{A}" represented by the following general formula (g-1), can be used.

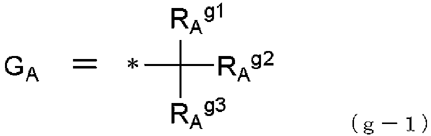

"R_{A}^{g1}" denotes an optionally substituted C₁₋₁₂ hydrocarbyl group in which any divalent carbon atom except the terminal positions may be replaced by -O-, -C(=O)-, - C(=O)O-, -OCO-, -CONH-, NHCO-, -S-, or -SO₂- (provided that adjacent divalent carbon atoms are not replaced at the same time).

R_{A}^{g1} preferably denotes a C₁₋₁₂ alkyl group, a C₃₋₁₂ alicyclic group, a C₆₋₁₂ aryl group, or a C₇₋₁₂ aralkyl group each optionally having a substituent and any divalent carbon atom except the terminal positions may be replaced by -O-, -C(=O)-, -C(=O)O-, -OCO-, -CONH-, NHCO-, -S-, or -SO₂- (provided that adjacent divalent carbon atoms are not replaced at the same time),
more preferably a C₁₋₁₂ alkyl group or a C₃₋₁₂ alicyclic group each optionally having a substituent and any divalent carbon atom except the terminal positions may be replaced by -O-, -C(=O)-, -C(=O)O-, -OCO-, -CONH-, NHCO-, -S-, or -SO₂- (provided that adjacent divalent carbon atoms are not replaced at the same time).

"R_{A}^{g2}" and "R_{A}^{g3}" each independently denote an optionally substituted C₁₋₁₂ hydrocarbyl group, and a divalent carbon atom at any position except the terminal positions may be substituted with -O-, -C(=O)-, -C(=O)O-, -OCO-, -CONH-, NHCO-, -S-, or -SO₂-(provided that adjacent divalent carbon atoms are not substituted at the same time), or R_{A}^{g2} and R_{A}^{g3} may be combined to form a C₃₋₁₈ alicyclic group or a 3- to 18-membered non-aromatic heterocyclic group, each optionally having a substituent, and a divalent carbon atom at any position except the terminal positions may be substituted with -O-, -C(=O)-, - C(=O)O-, -OCO-, -CONH-, NHCO-, -S-, or -SO₂- (provided that adjacent divalent carbon atoms are not substituted at the same time).

In R_{A}^{g2} and R_{A}^{g3}, preferably, R_{A}^{g2} and R_{A}^{g3} may be combined to form a C₃₋₁₈ alicyclic group or a 3- to 18-membered non-aromatic heterocyclic group, each optionally having a substituent, and a divalent carbon atom at any position except the terminal positions may be substituted with -O-, -C(=O)-, -C(=O)O-, -OCO-, -CONH-, NHCO-, -S-, or -SO₂- (provided that adjacent divalent carbon atoms are not substituted at the same time).

More preferably, the substituted C₃₋₁₈ alicyclic group or the 3- to 18-membered non-aromatic heterocyclic group, each optionally having a substituent, has a cyclopentane skeleton, a cyclohexane skeleton, a cycloheptane skeleton, a cyclooctane skeleton, a cyclononane skeleton, a cyclodecane skeleton, a cyclododecane skeleton, a cyclopentene skeleton, a cyclohexene skeleton, a cycloheptene skeleton, a cyclooctene skeleton, a cyclodecene skeleton, a norbornane skeleton, an adamantane skeleton, a tricyclodecane skeleton, a tetracyclododecane skeleton, a norbornene skeleton, or a tricyclodecene skeleton, and a divalent carbon atom at any position except the terminal positions may be substituted with -O-, -C(=O)-, -C(=O)O-, -OCO-, -CONH-, NHCO-, -S-, or -SO₂- (provided that adjacent divalent carbon atoms are not substituted at the same time).

A tertiary-carbon-type acid-dissociable group G_{A} represented by the general formula (g-1) is, for example, but not limited to, a t-butyl group, a t-amyl group, a 1,1-dimethylpropyl group, a 1-methyl-1-cyclopentyl group, a 1-ethyl-1-cyclopentyl group, a 1-methyl-1-cyclohexyl group, a 1-ethyl-1-cyclohexyl group, a 2-methyl-2-adamantyl group, a 2-ethyl-2-adamantyl group, a 1-(1-methoxy-2-methylpropan-2-yl)cyclopentyl group, a 1-(1-ethoxy-2-methylpropan-2-yl)cyclopentyl group, or the like.

### <<Tertiary-Carbon-Type Acid-Dissociable Group G_{A1} and G_{A2}>>

In the "tertiary-carbon-type acid-dissociable group G_{A}" represented by the general formula (g-1), the case where R_{A}^{g2} and R_{A}^{g3} may be combined to form a C₃₋₁₈ alicyclic group or a 3- to 18-membered non-aromatic heterocyclic group, each optionally having a substituent, is, for example, a tertiary-carbon-type acid-dissociable group G_{A1} represented by the following general formula (g-1-1), a tertiary-carbon-type acid-dissociable group G_{A2} represented by the following general formula (g-1-2), or the like.

### <<Tertiary-Carbon-Type Acid-Dissociable Group G_{A1} Represented by General Formula (g-1-1)>>

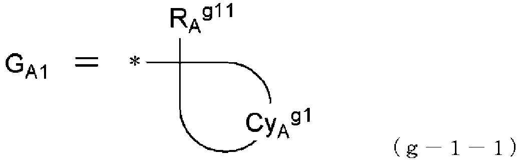

In the tertiary-carbon-type acid-dissociable group G_{A1} represented by the general formula (g-1-1), R_{A}^{g11} denotes a C₁₋₁₂ alkyl group, a C₃₋₁₂ alicyclic group, a C₆₋₁₂ aryl group, or a C₇₋₁₂ aralkyl group, each optionally having a substituent, and any divalent carbon atom except the terminal positions may be substituted with -O-, -C(=O)-, -C(=O)O-, -OCO-, - CONH-, NHCO-, -S-, or -SO₂- (provided that adjacent divalent carbon atoms are not substituted at the same time).

Cy_{A}^{g1}, together with the tertiary carbon atom, forms a C₃₋₁₈ alicyclic group or a 3- to 18-membered non-aromatic heterocyclic group, each optionally having a substituent, and any divalent carbon atom may be substituted with -O-, -C(=O)-, -C(=O)O-, -OCO-, -CONH-, NHCO-, -S-, or -SO₂- (provided that adjacent divalent carbon atoms are not substituted at the same time).

Preferably, Cy_{A}^{g1}, together with the tertiary carbon atom, forms a cyclopentane skeleton, a cyclohexane skeleton, a cycloheptane skeleton, a cyclooctane skeleton, a cyclononane skeleton, a cyclodecane skeleton, a cyclododecane skeleton, a cyclopentene skeleton, a cyclohexene skeleton, a cycloheptene skeleton, a cyclooctene skeleton, a cyclodecene skeleton, a norbornane skeleton, an adamantane skeleton, a tricyclodecane skeleton, a tetracyclododecane skeleton, a norbornene skeleton, or a tricyclodecene skeleton, each optionally having a substituent, and a divalent carbon atom at any position except the terminal positions may be substituted with -O-, -C(=O)-, -C(=O)O-, -OCO-, -CONH-, NHCO-, -S-, or -SO₂- (provided that adjacent divalent carbon atoms are not substituted at the same time).

The tertiary-carbon-type acid-dissociable group G_{A1} represented by the general formula (g-1-1) is, for example, the following tertiary-carbon-type acid-dissociable group.

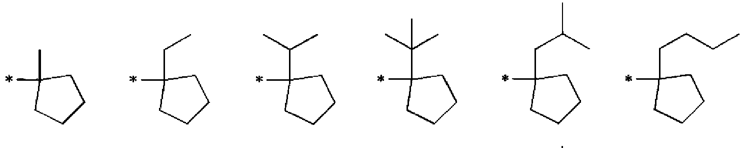

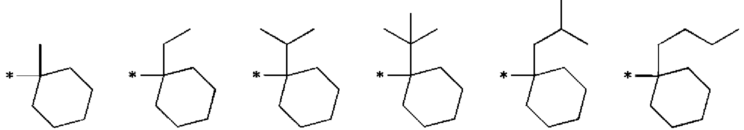

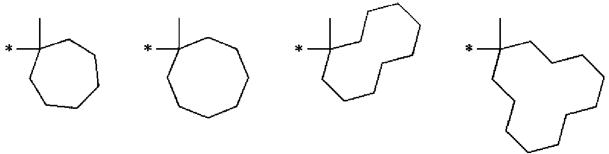

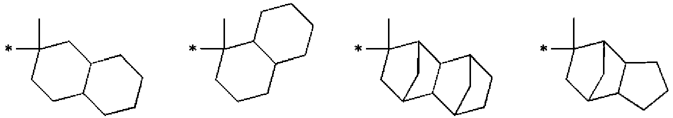

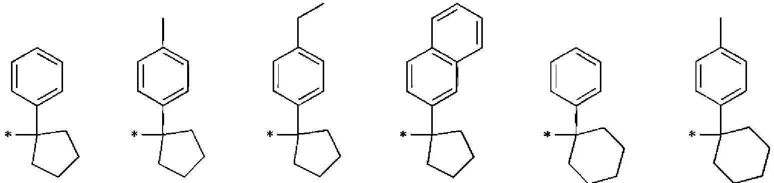

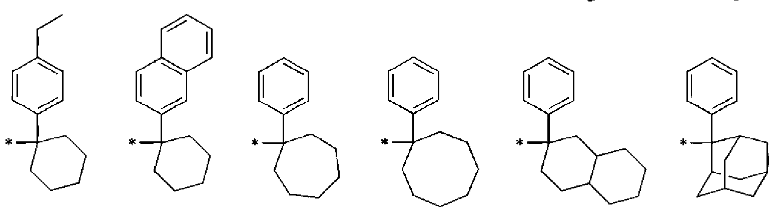

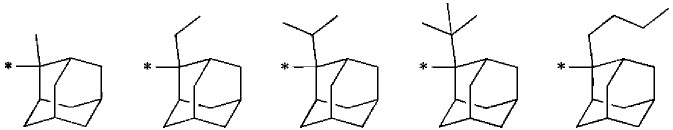

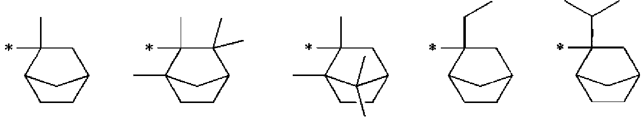

### <<Tertiary-Carbon-Type Acid-Dissociable Group G_{A2} Represented by General Formula (g-1-2)>>

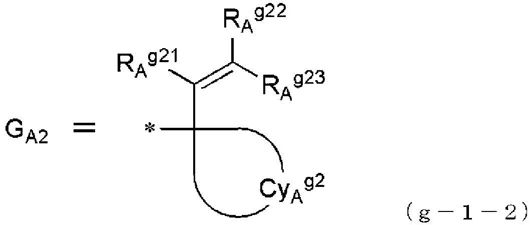

In the general formula (g-1-2), R_{A}^{g21} to R_{A}^{g23} each independently denote a hydro group or an optionally substituted C₁₋₁₂ hydrocarbyl group, any divalent carbon atom except the terminal positions may be substituted with -O-, -C(=O)-, -C(=O)O-, -OCO-, -CONH-, NHCO-, -S-, or -SO₂- (provided that adjacent divalent carbon atoms are not substituted at the same time), and R_{A}^{g21} and R_{A}^{g22}, and/or R_{A}^{g22} and R_{A}^{g23} may be directly bonded to each other by a single bond or may be bonded to each other via a divalent linker such as, -O-, - S-, -C(=O)-, -S(=O)-, -S(=O)₂-, -C(=O)O-, or a C₁₋₃ alkylene group, to form a ring.

The case where R_{A}^{g21} and R_{A}^{g22}, and/or R_{A}^{g22} and R_{A}^{g23} form a ring together with a carbon atom contained in the ethylenically unsaturated double bond is, for example, a case where they forms a cyclopentenyl group, a cyclohexenyl group, a cyclopentylidene ethenyl group, a cyclohexylidene ethenyl group, or the like, each optionally having a substituent.

Cy_{A}^{g2}, together with the tertiary carbon atom, forms a C₃₋₁₈ alicyclic group or a 3- to 18-membered non-aromatic heterocyclic group, each optionally having a substituent, and any divalent carbon atom may be substituted with -O-, -C(=O)-, -C(=O)O-, -OCO-, -CONH-, NHCO-, -S-, or -SO₂- (provided that adjacent divalent carbon atoms are not substituted at the same time).

Preferably, Cy_{A}^{g2}, together with the tertiary carbon atom, forms a cyclopentane skeleton, a cyclohexane skeleton, a cycloheptane skeleton, a cyclooctane skeleton, a cyclononane skeleton, a cyclodecane skeleton, a cyclododecane skeleton, a cyclopentene skeleton, a cyclohexene skeleton, a cycloheptene skeleton, a cyclooctene skeleton, a cyclodecene skeleton, a norbornane skeleton, an adamantane skeleton, a tricyclodecane skeleton, a tetracyclododecane skeleton, a norbornene skeleton, or a tricyclodecene skeleton, each optionally having a substituent, and a divalent carbon atom at any position except the terminal positions may be substituted with -O-, -C(=O)-, -C(=O)O-, -OCO-, -CONH-, NHCO-, -S-, or -SO₂- (provided that adjacent divalent carbon atoms are not substituted at the same time).

The tertiary-carbon-type acid-dissociable group G_{A2} represented by the general formula (g-1-2) is, for example, the following tertiary-carbon-type acid-dissociable group.

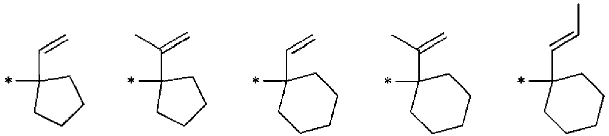

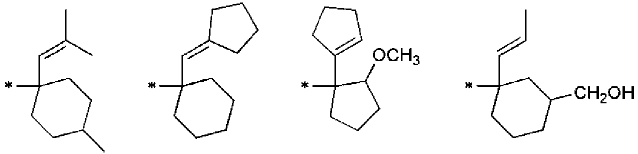

The phrase "a hydroxy group or a carboxy group each having a protective group", as used herein, refers to a hydroxy group (including a phenolic hydroxy group) or a carboxy group in which the hydroxy group or the carboxy group is protected by an ether protective group, a silyl ether protective group, an acetal protective group, an acyl protective group, an oxycarbonyl (alkyl) protective group, an aminocarbonyl protective group, or the like, or refers to a hydroxy group (including a phenolic hydroxy group) or a carboxy group each having an acid-dissociable group.

The ether protective group is, for example, but not limited to, a methyl group, a benzyl group, a p-methoxybenzyl group, a t-butyl group, a triphenylmethyl group, a p-methoxyphenyldiphenylmethyl group, a di(p-methoxyphenyl)phenylmethyl group, or the like.

The silyl ether protective group is, for example, but not limited to, a t-butyldimethylsilyl group (TBS), a triisopropylsilyl group (TIPS), a trimethylsilyl group (TMS), a triethylsilyl group (TES), a t-butyldiphenylsilyl group (TBDPS), or the like.

The acetal protective group is, for example, but not limited to, a methoxymethyl group, an ethoxyethyl group, a 2-tetrahydropyranyl group (THP), a methoxyethoxymethyl group, or the like.

The acyl protective group is, for example, but not limited to, an acetyl group, a pivaloyl group, a benzoyl group, or the like.

The oxycarbonyl (alkyl) protective group is, for example, but not limited to, a t-butoxycarbonyl group or the like.

The aminocarbonyl protective group is, for example, but not limited to, a dimethylaminocarbonyl group, a diethylaminocarbonyl group, a diisopropylaminocarbonyl group, an N-phenyl-N-methyl-aminocarbonyl group, or the like.

The protective group is, for example, a protective group P for a hydroxy group or a carboxy group represented by the following general formula (P-1) or (P-2).

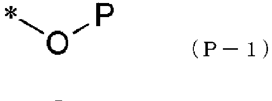

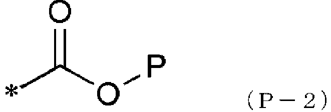

The protective group P is not particularly limited and may be a known and commonly used protective group. The protective group P other than the "acid-dissociable group" is, for example, an "acetal protective group P_{A}" represented by the following general formula (p-1), a "silyl ether protective group P_{B}" represented by the following general formula (p-2), an "aminocarbonyl protective group P_{C}" represented by the following general formula (p-3), or the like. Each of them will be sequentially described below.

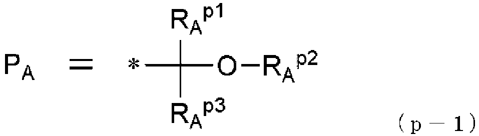

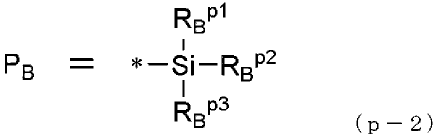

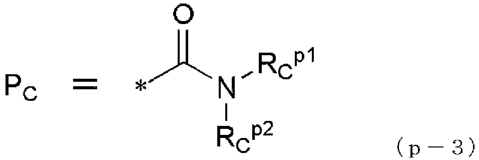

### <Acetal Protective Group P_{A}>

The protective group P may be a protective group in which an oxygen atom is bonded to a carbon atom directly bonded to an oxygen atom of a hydroxy group, a phenolic hydroxy group, or a carboxy group, such as the "acetal protective group P_{A}" represented by the following general formula (p-1).

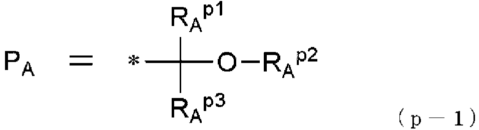

"R_{A}^{p1}" and "R_{A}^{p3}" each independently denote a hydro group or an optionally substituted C₁₋₁₂ hydrocarbyl group, and any divalent carbon atom except the terminal positions may be substituted with -O-, -C(=O)-, -C(=O)O-, -OCO-, -CONH-, NHCO-, -S-, or -SO₂- (provided that adjacent divalent carbon atoms are not substituted at the same time).

"R_{A}^{p2}" denotes an optionally substituted C₁₋₁₂ hydrocarbyl group, and any divalent carbon atom except the terminal positions may be substituted with -O-, -C(=O)-, -C(=O)O-, -OCO-, -CONH-, NHCO-, -S-, or -SO₂- (provided that adjacent divalent carbon atoms are not substituted at the same time).

Preferably, R_{A}^{p1} and R_{A}^{p3} denote a hydro group, or a C₁₋₁₂ alkyl group or a C₃₋₁₂ alicyclic group, the alkyl group and the alicyclic group each optionally having a substituent, and any divalent carbon atom except the terminal positions may be substituted with -O-, - C(=O)-, -C(=O)O-, -OCO-, -CONH-, NHCO-, -S-, or -SO₂- (provided that adjacent divalent carbon atoms are not substituted at the same time),
more preferably a hydro group, or a C₁₋₆ alkyl group or a C₃₋₈ alicyclic group, the alkyl group and the alicyclic group each optionally having a substituent, and any divalent carbon atom except the terminal positions may be substituted with -O-, -C(=O)-, -C(=O)O-, -OCO-, -CONH-, NHCO-, -S-, or -SO₂- (provided that adjacent divalent carbon atoms are not substituted at the same time).

Preferably, R_{A}^{p2} denotes a C₁₋₁₂ alkyl group or a C₃₋₁₂ alicyclic group, the alkyl group and the alicyclic group each optionally having a substituent, and any divalent carbon atom except the terminal positions may be substituted with -O-, -C(=O)-, -C(=O)O-, -OCO-, - CONH-, NHCO-, -S-, or -SO₂- (provided that adjacent divalent carbon atoms are not substituted at the same time),
more preferably a C₁₋₆ alkyl group or a C₃₋₈ alicyclic group, the alkyl group and the alicyclic group each optionally having a substituent, and any divalent carbon atom except the terminal positions may be substituted with -O-, -C(=O)-, -C(=O)O-, -OCO-, -CONH-, NHCO-, -S-, or -SO₂- (provided that adjacent divalent carbon atoms are not substituted at the same time).

The acetal protective group P_{A} represented by the general formula (p-1) is, for example, a methoxymethoxy group, an ethoxymethoxy group, a n-propoxymethoxy group, a n-butoxymethoxy group, a 2,2-dimethylpropoxymethoxy group, a 2,2,-dimethylbutoxymethoxy group, a cyclohexyloxymethoxy group, a 1-ethoxyethoxy group, a 1-n-butoxyethoxy group, a 1-cyclohexyloxyethoxy group, or the like.

### <Silyl Ether Protective Group P_{B}>

The protective group P may be a protective group in which a silicon atom is directly bonded to an oxygen atom of a hydroxy group, a phenolic hydroxy group, or a carboxy group, such as a "silyl ether protective group P_{B}" represented by the following general formula (p-2).

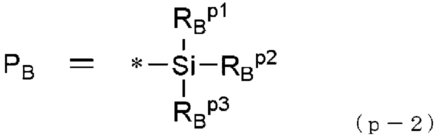

"R_{B}^{p1}" to "R_{B}^{p3}" each independently denote an optionally substituted C₁₋₁₂ hydrocarbyl group in which any divalent carbon atom except the terminal positions may be replaced by -O-, -C(=O)-, -C(=O)O-, -OCO-, -CONH-, NHCO-, -S-, or -SO₂- (provided that adjacent divalent carbon atoms are not replaced at the same time).

R_{B}^{p1} to R_{B}^{p3} preferably denote a C₁₋₁₂ alkyl group or a C₃₋₁₂ alicyclic group each optionally having a substituent and any divalent carbon atom except the terminal positions may be replaced by -O-, -C(=O)-, -C(=O)O-, -OCO-, -CONH-, NHCO-, -S-, or -SO₂-(provided that adjacent divalent carbon atoms are not replaced at the same time),
more preferably a C₁₋₆ alkyl group or a C₃₋₈ alicyclic group each optionally having a substituent and any divalent carbon atom except the terminal positions may be replaced by -O-, -C(=O)-, -C(=O)O-, -OCO-, -CONH-, NHCO-, -S-, or -SO₂- (provided that adjacent divalent carbon atoms are not replaced at the same time).

The silyl ether protective group P_{B} represented by the general formula (p-2) is, for example, but not limited to, a t-butyldimethylsilyl group (TBS), a triisopropylsilyl group (TIPS), a trimethylsilyl group (TMS), a triethylsilyl group (TES), a t-butyldiphenylsilyl group (TBDPS), or the like.

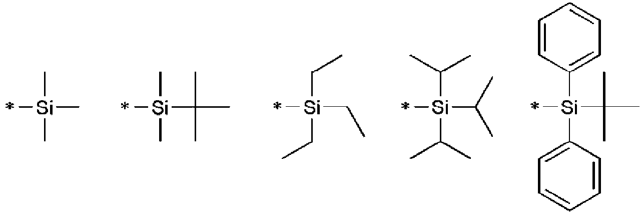

### <Aminocarbonyl Protective Group P_{C}>

The protective group P may be a protective group in which an aminocarbonyl group is bonded to an oxygen atom of a hydroxy group, a phenolic hydroxy group, or a carboxy group, such as an "aminocarbonyl protective group P_{C}" represented by the following general formula (p-3).

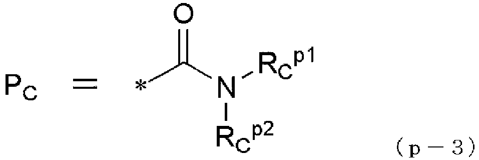

"R_{C}^{p1}" and "R_{C}^{p2}" each independently denote a hydro group, or an optionally substituted C₁₋₁₂ hydrocarbyl group in which any divalent carbon atom except the terminal positions may be replaced by -O-, -C(=O)-, -C(=O)O-, -OCO-, -CONH-, NHCO-, -S-, or - SO₂- (provided that adjacent divalent carbon atoms are not replaced at the same time).

Preferably, R_{C}^{p1} and R_{C}^{p2} denote a hydro group, or a C₁₋₁₂ alkyl group or a C₃₋₁₂ alicyclic group, the alkyl group and the alicyclic group each optionally having a substituent, and any divalent carbon atom except the terminal positions may be substituted with -O-, - C(=O)-, -C(=O)O-, -OCO-, -CONH-, NHCO-, -S-, or -SO₂- (provided that adjacent divalent carbon atoms are not substituted at the same time),
more preferably a hydro group, or a C₁₋₆ alkyl group or a C₃₋₈ alicyclic group, the alkyl group and the alicyclic group each optionally having a substituent, and any divalent carbon atom except the terminal positions may be substituted with -O-, -C(=O)-, -C(=O)O-, -OCO-, -CONH-, NHCO-, -S-, or -SO₂- (provided that adjacent divalent carbon atoms are not substituted at the same time).

The aminocarbonyl protective group P_{C} represented by the general formula (p-3) is, for example, but not limited to, a dimethylaminocarbonyl group, a diethylaminocarbonyl group, a diisopropylaminocarbonyl group, an N-phenyl-N-methyl-aminocarbonyl group, or the like.

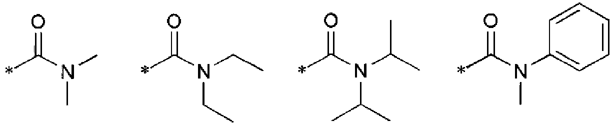

The phrase "optionally substituted" or "optionally having a substituent", as used herein, is not particularly limited provided that it is chemically acceptable and has the advantages of the present invention.

The "substituent" is, for example, (1) a fluorine atom, (2) a chlorine atom, (3) a bromine atom, (4) a haloalkyl group, (5) a hydroxy group, (6) a thiol group, (7) a nitro group, (8) a cyano group, (9) a carboxy group, (10) an amino group, (11) a sulfo group, (12) a vinyl group, (13) an allyl group, (14) a (meth)acryloyl group, (15) a (meth)acryloyloxy group, (16) a (meth)acrylamide group, (17) a styryl group, (18) an epoxy group, (19) a glycidyl group, (20) an amide group, (21) a hydroxy group or a carboxy group each having a protective group, or (22) a C₁₋₁₈ hydrocarbyl group, a C₁₋₁₈ hydrocarbyloxy group, a C₁₋₁₈ hydrocarbylcarbonyl group, a C₁₋₁₈ hydrocarbylcarbonyloxy group, a C₁₋₁₈ hydrocarbyloxycarbonyl group, a C₁₋₁₈ hydrocarbyloxycarbonyloxy group, a C₁₋₁₈ hydrocarbylamino group, a di-C₁₋₁₈ hydrocarbylamino group, a C₁₋₁₈ hydrocarbylaminocarbonyl group, a di-C₁₋₁₈ hydrocarbylaminocarbonyl group, a C₁₋₁₈ hydrocarbylcarbonylamino group, a C₁₋₁₈ hydrocarbylaminocarbonyloxy group, a di-C₁₋₁₈ hydrocarbylaminocarbonyloxy group, a C₁₋₁₈ hydrocarbylaminocarbonylamino group, a di-C₁₋₁₈ hydrocarbylaminocarbonylamino group, a C₁₋₁₈ hydrocarbyloxycarbonylamino group, a C₁₋₁₈ hydrocarbylthio group, a C₁₋₁₈ hydrocarbylsulfinyl group, or a C₁₋₁₈ hydrocarbylsulfonyl group, in which at least part of hydrogen atoms may be substituted with (1) to (21), and a divalent carbon atom at any position of these substituents except the terminal positions may be substituted with -O-, -C(=O)-, -C(=O)O-, -OCO-, -CONH-, - NHCO-, -NH(C=O)O-, -S-, or -SO₂- (provided that adjacent divalent carbon atoms are not substituted at the same time), or the like.

### [1. Polyacid Salt or Mixture Thereof]

A polyacid salt or a mixture thereof according to the present embodiment is represented by the following general formula (I).

(A^{m+})ₐ(Bⁿ⁺)_{b}(C^{(am+bn)-}) (I)

[wherein
A^{m+} each independently denotes H⁺, a metal ion, or an ammonium ion,
Bⁿ⁺ each independently denotes an organic quaternary ammonium cation, an organic quaternary ammonium dication, or a pyridinium cation, each having two or more ethylenically unsaturated double bonds,
C^{(am+bn)-} denotes a polyacid anion, and
m denotes an integer in the range of 1 to 5, n denotes an integer of 1 or 2, a denotes a real number, and b denotes a real number greater than 0]

### [2. Polyacid Anion]

A polyacid anion according to the present embodiment is composed of an isopolyacid anion or a heteropolyacid anion.

### [2-1. Isopolyacid Anion]

The isopolyacid anion is, for example, but is not limited to, an isopolyoxomolybdate anion, an isopolyoxotungstate anion, an isopolyoxovanadate anion, an isopolyoxoniobate anion, an isopolyoxotantalate anion, or the like.

The isopolyoxomolybdate anion is, for example, [MoO₄]²⁻, [Mo₇O₂₄]⁶⁻, [Mo₈O₂₆]⁴⁻, or the like. The isopolyoxotungstate anion is, for example, [W₄O₁₃]²⁻, [W₅O₁₆]²⁻, [W₆O₁₉]²⁻, [W₇O₂₂]²⁻, [W₇O₂₄]⁶⁻, [H_{z}W₁₂O₄₀]^{-(8-z)} (wherein z denotes an integer in the range of 1 to 4), [W₁₀O₃₂]⁴⁻, [H₄W₁₁O₃₈]⁶⁻, [H₇W₁₁O₄₀]⁷⁻, [HW₅O₁₉]⁷⁻, [H₃W₁₁O₂₂]⁵⁻, or the like. The isopolyoxovanadate anion is, for example, [V₄O₁₂]⁴⁻, [V₁₀O₂₈]⁶⁻, or the like, the isopolyoxoniobate anion is, for example, [Nb₆O₁₉]⁸⁻, [Nb₁₀O₂₈]⁶⁻, or the like, and the isopolyoxotantalate anion is, for example, [Ta₆O₁₉]⁸⁻, [Ta₈O₂₁]²⁻, or the like.

The isopolyoxomolybdate anion, the isopolyoxotungstate anion, the isopolyoxovanadate anion, the isopolyoxoniobate anion, and the isopolyoxotantalate anion include various isomers and the like.

The isopolyoxotungstate anion is preferably [W₄O₁₃]²⁻, [W₅O₁₆]²⁻, [W₆O₁₉]²⁻, [W₇O₂₂]²⁻, [W₇O₂₄]⁶⁻, [W₁₀O₃₂]⁴⁻, [H_{z}W₁₂O₄₀]^{-(8-z)} (z denotes an integer in the range of 1 to 4), [H₄W₁₁O₃₈]⁶⁻, [H₇W₁₁O₄₀]⁷⁻, [HW₅O₁₉]⁷⁻, [H₃W₁₁O₂₂]⁵⁻, [H₄W₂₂O₇₄]¹²⁻, or [H₁₀W₃₄O₁₁₆]¹⁸⁻, more preferably [W₇O₂₄]⁶⁻, [W₁₀O₃₂]⁴⁻, or [H_{z}W₁₂O₄₀]^{-(8-z)} (z denotes an integer in the range of 1 to 4), still more preferably [W₇O₂₄]⁶⁻, [W₁₀O₃₂]⁴⁻, or [H₂W₁₂O₄₀]⁶⁻.

### [2-2. Heteropolyacid Anion]

The heteropolyacid anion is, for example, but is not limited to, a heteropolyoxomolybdate anion, a heteropolyoxotungstate anion, a heteropolyoxovanadate anion, a heteropolyoxoniobate anion, a heteropolyoxotantalate anion, or the like.

The polyatom of the heteropolyacid anion is preferably Mo, W, V, Nb, or Ta, more preferably Mo or W.

The heteropolyoxomolybdate anion is, for example, a phosphomolybdate anion, a silicomolybdate anion, a boromolybdate anion, a phosphotungstomolybdate anion, a cobalt molybdate anion, an arsenic molybdate anion, a germanium molybdate anion, or the like. The heteropolyoxotungstate anion is, for example, a phosphotungstate anion, a silicotungstate anion, a borotungstate anion, a cobalt tungstate anion, an arsenic tungstate anion, a germanium tungstate anion, or the like. The heteropolyoxovanadate anion is, for example, a phosphomolybdovanadate anion, a phosphomolybdotungstomolybdate anion, a boromolybdovanadate anion, a boromolybdotungstovanadate anion, or the like.

The heteroatom of the heteropolyacid anion is preferably P, Si, Ge, B, S, Co, or As, more preferably P, Si, B, S, or Ge.

The heteropolyoxomolybdate anion, the heteropolyoxotungstate anion, the heteropolyoxovanadate anion, and the like include a Keggin type, a Dawson type, an Anderson type, and isomers thereof.

The heteropolyoxotungstate anion is preferably [PW₁₂O₄₀]³⁻, [SiW₁₂O₄₀]⁴⁻, [BW₁₂O₄₀]⁵⁻, [SW₁₂O₄₀]²⁻, [P₂W₁₈O₆₂]⁶⁻, [Si₂W₁₈O₆₂]⁸⁻, and [S₂W₁₈O₆₂]⁴⁻.

### [3. Cation Moiety of Polyacid Salt]

The polyacid salt or a mixture thereof according to the present embodiment is represented by the following general formula (I).

(A^{m+})ₐ(Bⁿ⁺)_{b}(C^{(am+bn)-}) (I)

[wherein
A^{m+} each independently denotes H⁺, a metal ion, or an ammonium ion,
Bⁿ⁺ each independently denotes an organic quaternary ammonium cation, an organic quaternary ammonium dication, or a pyridinium cation, each having two or more ethylenically unsaturated double bonds,
C^{(am+bn)-} denotes a polyacid anion, and
m denotes an integer in the range of 1 to 5, n denotes an integer of 1 or 2, a denotes a real number, and b denotes a real number greater than 0]

A^{m+} and Bⁿ⁺ correspond to the cation moieties of the polyacid salt. In the following, A^{m+} is also referred to as "another counter cation".

In the following description, "Bⁿ⁺" is described first, and the other counter cation "A^{m+}" is described thereafter.

### [3-1. Organic Quaternary Ammonium Cation, Organic Quaternary Ammonium Dication, or Pyridinium Cation (Bⁿ⁺)]

### [3-1-1. Organic Quaternary Ammonium Cation]

An organic quaternary ammonium cation, an organic quaternary ammonium dication, or a pyridinium cation, each having two or more ethylenically unsaturated double bonds, according to the present embodiment is not particularly limited and may be a known and commonly used one. The ethylenically unsaturated double bond may be derived from, for example, a vinyl group, a (meth)acryloyl group, a (meth)acryloyloxy group, a (meth)acrylamide group, or a styryl group.

The organic quaternary ammonium cation having two or more ethylenically unsaturated double bonds according to the present embodiment is, for example, an organic quaternary ammonium cation represented by the following general formula (II). 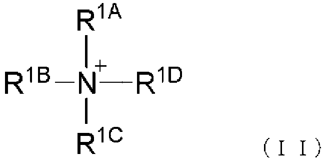 [wherein
R^{1A} to R^{1D} each independently denote a C₁₋₁₈ hydrocarbyl group, a 3- to 18-membered non-aromatic heterocyclic group, or a 5- to 18-membered aromatic heterocyclic group, the hydrocarbyl group and the heterocyclic groups each optionally having a substituent,
a divalent carbon atom at any position of R^{1A} to R^{1D} except the terminal positions may be substituted with -O-, -C(=O)-, -C(=O)O-, -OCO-, -CONH-, -NHCO-, -NH(C=O)O-, -S-, or -SO₂- (provided that adjacent divalent carbon atoms are not substituted at the same time),
a hydrogen atom in R^{1A} to R^{1D} may be substituted with (a) a fluorine atom, (b) a chlorine atom, (c) a bromine atom, (d) a haloalkyl group, (e) a hydroxy group, (f) a thiol group, (g) a nitro group, (h) a cyano group, (i) a carboxy group, (j) an amino group, (k) a sulfo group, (l) a vinyl group, (m) an allyl group, (n) a (meth)acryloyl group, (o) a (meth)acryloyloxy group, (p) a (meth)acrylamide group, (q) a styryl group, (r) an epoxy group, (s) a glycidyl group, (t) an amide group, (u) a hydroxy group or a carboxy group each having a protective group, or (v) a C₁₋₁₈ hydrocarbyl group, a C₁₋₁₈ hydrocarbyloxy group, a C₁₋₁₈ hydrocarbylcarbonyl group, a C₁₋₁₈ hydrocarbylcarbonyloxy group, a C₁₋₁₈ hydrocarbyloxycarbonyl group, a C₁₋₁₈ hydrocarbyloxycarbonyloxy group, a C₁₋₁₈ hydrocarbylamino group, a di-C₁₋₁₈ hydrocarbylamino group, a C₁₋₁₈ hydrocarbylaminocarbonyl group, a di-C₁₋₁₈ hydrocarbylaminocarbonyl group, a C₁₋₁₈ hydrocarbylcarbonylamino group, a C₁₋₁₈ hydrocarbylaminocarbonyloxy group, a di-C₁₋₁₈ hydrocarbylaminocarbonyloxy group, a C₁₋₁₈ hydrocarbylaminocarbonylamino group, a di-C₁₋₁₈ hydrocarbylaminocarbonylamino group, a C₁₋₁₈ hydrocarbyloxycarbonylamino group, or a C₁₋₁₈ hydrocarbylthio group, in which at least part of hydrogen atoms may be substituted with (a) to (u), and a divalent carbon atom at any position of these substituents except the terminal positions may be substituted with -O-, -C(=O)-, -C(=O)O-, -OCO-, -CONH-, - NHCO-, -NH(C=O)O-, -S-, or -SO₂- (provided that adjacent divalent carbon atoms are not substituted at the same time),
at least one hydrogen atom in R^{1A} and R^{1B} is substituted with (l) a vinyl group, (m) an allyl group, (n) a (meth)acryloyl group, (o) a (meth)acryloyloxy group, (p) a (meth)acrylamide group, or (q) a styryl group, and
any two of R^{1A} to R^{1D} may be directly bonded to each other by a single bond or may be bonded to each other via a divalent linking group -S-, -C(=O)-, -S(=O)-, -S(=O)₂-, - C(=O)O-, -C(=O)NH-, or a C₁₋₃ alkylene group to form a ring together with the nitrogen atom in the formula (II)]

Specific examples of R^{1A} and R^{1B} include, but are not limited to, a vinyl group, an allyl group, a propenyl group, a butenyl group, a pentenyl group, a hexenyl group, a heptenyl group, a (meth)acryloylethyl group, a (meth)acryloylpropyl group, a (meth)acryloyloxyethyl group, a (meth)acryloyloxypropyl group, a (meth)acryloylaminoethyl group, a (meth)acryloylaminopropyl group, a styryl group, and the like.

As an organic quaternary ammonium cation having two or more ethylenically unsaturated double bonds according to another embodiment, an organic quaternary ammonium cation represented by the following general formula (III) can be used, for example. 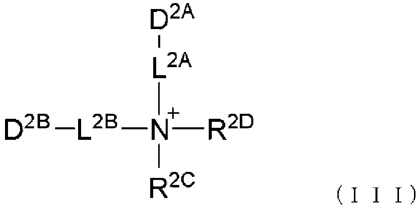 [wherein
L^{2A} and L^{2B} each independently denote an optionally substituted C₁₋₁₈ hydrocarbylene group,
a divalent carbon atom at any position of L^{2A} and L^{2B} including the terminal positions may be substituted with -O-, -C(=O)-, -C(=O)O-, -OCO-, -CONH-, -NHCO-, -NH(C=O)O-, -S-, or -SO₂- (provided that adjacent divalent carbon atoms are not substituted at the same time),
D^{2A} and D^{2B} each independently denote a vinyl group, a (meth)acryloyl group, a (meth)acryloyloxy group, a (meth)acrylamide group, or a styryl group,
R^{2C} and R^{2D} each independently denote a C₁₋₁₈ hydrocarbyl group, a 3- to 18-membered non-aromatic heterocyclic group, or a 5- to 18-membered aromatic heterocyclic group, the hydrocarbyl group and the heterocyclic groups each optionally having a substituent,
a divalent carbon atom at any position of R^{2C} and R^{2D} except the terminal positions may be substituted with -O-, -C(=O)-, -C(=O)O-, -OCO-, -CONH-, -NHCO-, -NH(C=O)O-, -S-, or -SO₂- (provided that adjacent divalent carbon atoms are not substituted at the same time),
a hydrogen atom in L^{2A}, L^{2B}, R^{2C}, and R^{2D} may be substituted with (a) a fluorine atom, (b) a chlorine atom, (c) a bromine atom, (d) a C₁₋₃ haloalkyl group, (e) a hydroxy group, (f) a thiol group, (g) a nitro group, (h) a cyano group, (i) a carboxy group, (j) an amino group, (k) a sulfo group, (l) a vinyl group, (m) an allyl group, (n) a (meth)acryloyl group, (o) a (meth)acryloyloxy group, (p) a (meth)acrylamide group, (q) a styryl group, (r) an epoxy group, (s) a glycidyl group, (t) an amide group, or (u) a hydroxy group or a carboxy group each having a protective group, or (v) a C₁₋₁₈ hydrocarbyl group, a C₁₋₁₈ hydrocarbyloxy group, a C₁₋₁₈ hydrocarbylcarbonyl group, a C₁₋₁₈ hydrocarbylcarbonyloxy group, a C₁₋₁₈ hydrocarbyloxycarbonyl group, a C₁₋₁₈ hydrocarbyloxycarbonyloxy group, a C₁₋₁₈ hydrocarbylamino group, a di-C₁₋₁₈ hydrocarbylamino group, a C₁₋₁₈ hydrocarbylaminocarbonyl group, a di-C₁₋₁₈ hydrocarbylaminocarbonyl group, a C₁₋₁₈ hydrocarbylcarbonylamino group, a C₁₋₁₈ hydrocarbylaminocarbonyloxy group, a di-C₁₋₁₈ hydrocarbylaminocarbonyloxy group, a C₁₋₁₈ hydrocarbylaminocarbonylamino group, a di-C₁₋₁₈ hydrocarbylaminocarbonylamino group, a C₁₋₁₈ hydrocarbyloxycarbonylamino group, or a C₁₋₁₈ hydrocarbylthio group, in which at least part of hydrogen atoms may be substituted with (a) to (u), and a divalent carbon atom at any position of these substituents except the terminal positions may be substituted with -O-, -C(=O)-, -C(=O)O-, -OCO-, -CONH-, - NHCO-, -NH(C=O)O-, -S-, or -SO₂- (provided that adjacent divalent carbon atoms are not substituted at the same time),
any two of L^{2A}, L^{2B}, R^{2C}, and R^{2D} may be directly bonded to each other by a single bond or may be bonded to each other via a divalent linking group -S-, -C(=O)-, -S(=O)-, - S(=O)₂-, -C(=O)O-, -C(=O)NH-, or a C₁₋₃ alkylene group to form a ring together with the nitrogen atom in the formula (III)]

Specific examples of the organic quaternary ammonium cations represented by the general formulae (II) and (III) include, but are not limited to, a diallyldimethylammonium cation, a tetraallylammonium cation, a triallylmethylammonium cation, a triallyl-n-pentylammonium cation, a triallylphenylammonium cation, a triallyl(2-carboxyethyl)ammonium cation, a triallyl(2-sulfoethyl)ammonium cation, a triallyl(3-hydroxypropyl)ammonium cation, a triallyl(3-hydroxy-(2-hydroxymethyl)propyl)ammonium cation, a triallyl(2,3-dihydroxypropyl)ammonium cation, a triallyl(methoxyethyl)ammonium cation, a triallyl(3,3,3-trifluoropropyl)ammonium cation, a triallyl(2-trifluoromethylphenyl)ammonium cation, a triallyl(2,4-bis(trifluoromethyl)benzyl)ammonium cation, a triallyl(2,4-bis(trifluoromethyl)phenyl)ammonium cation, a triallyl(4-vinylbenzyl)ammonium cation, a triallyl(3,5-bis(vinyl)benzyl)ammonium cation, a triallyl(acryloylmethyl)ammonium cation, a triallyl(3-acrylamidopropyl)ammonium cation, a triallyl(3-N-methyl-acryloylamidopropyl)ammonium cation, a triallyl(N,N-diallylaminocarbonylmethyl)ammonium cation, a dimethyldi(2-N-methylacrylamidoethyl)ammonium cation, a di(3-acrylamidopropyl)dimethylammonium cation, a di(2-methacryloyloxyethyl)dimethylammonium cation, a dimethyldivinylammonium cation, and the like.

The organic quaternary ammonium cations having two or more ethylenically unsaturated double bonds are, for example, the following organic quaternary ammonium cations.

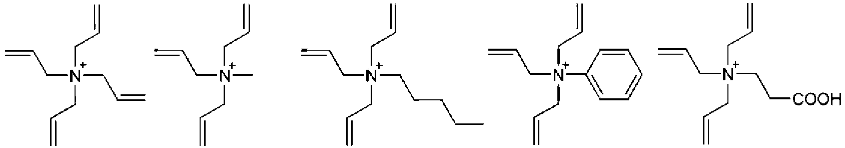

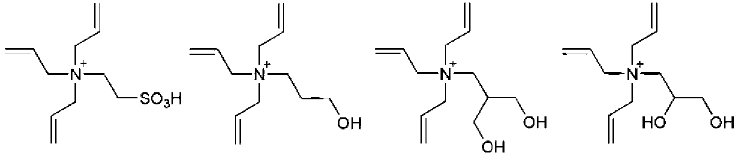

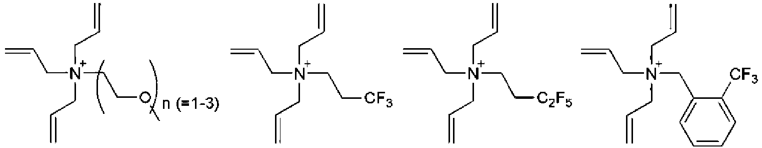

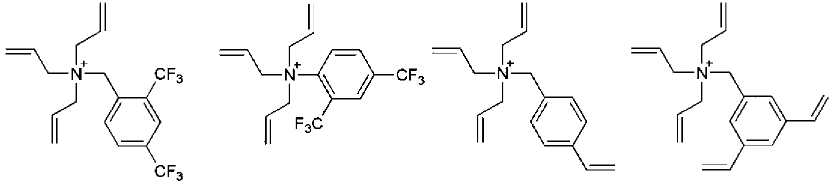

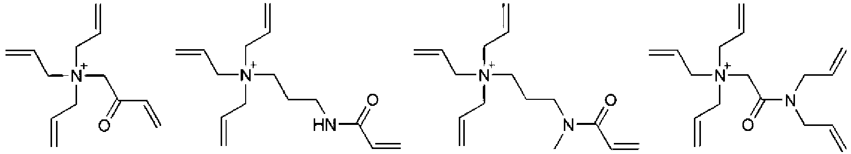

When R^{1A} and R^{1B} in the organic quaternary ammonium cation represented by the general formula (II) have one or more (meth)acryloyloxy groups, and when D^{2A} and D^{2B} in the organic quaternary ammonium cation represented by the general formula (III) have one or more (meth)acryloyloxy groups, specific examples thereof include, but are not limited to, a tris(2-acryloyloxyethyl)methylammonium cation, a (tris(2-acryloyloxyethyl))n-butylammonium cation, a (tris(2-acryloyloxyethyl))isopentylammonium cation, a (tris(2-acryloyloxyethyl))benzylammonium cation, a tris(2-acryloyloxyethyl)phenylammonium cation, a tris(2-acryloyloxyethyl)(3-carboxypropyl)ammonium cation, a tris(2-acryloyloxyethyl)(3-sulfopropyl)ammonium cation, a tris(2-acryloyloxyethyl)(2-methoxyethyl)ammonium cation, a tris(2-acryloyloxyethyl)(3-hydroxypropyl)ammonium cation, a tris(2-acryloyloxyethyl)(3-hydroxy-(2-hydroxymethyl)propyl)ammonium cation, a tris(2-acryloyloxyethyl)(2,3-dihydroxypropyl)ammonium cation, a tris(2-acryloyloxyethyl)(3,3,4,4,4-pentafluorobutyl)ammonium cation,
a tris(2-acryloyloxyethyl)(4-trifluoromethylbenzyl)ammonium cation, a tris(2-acryloyloxyethyl)(2-trifluoromethylbenzyl)ammonium cation, a tris(2-acryloyloxyethyl)(2,4-bis(trifluoromethyl)benzyl)ammonium cation, a tris(2-acryloyloxyethyl)(2-trifluoromethylphenyl)ammonium cation, a tris(2-acryloyloxyethyl)(2,4-bis(trifluoromethyl)phenyl)ammonium cation, a tris(2-acryloyloxyethyl)allylammonium cation, a tris(2-acryloyloxyethyl)(4-vinylbenzyl)ammonium cation, a tris(2-acryloyloxyethyl)(3,5-diallylbenzyl)ammonium cation, a tris(2-acryloyloxyethyl)(3-acryloyloxypropyl)ammonium cation, a tris(2-acryloyloxyethyl)(3-acrylamidopropyl)ammonium cation, a tris(2-acryloyloxyethyl)(3-N-methylacrylamidopropyl)ammonium cation, a tris(2-acryloyloxyethyl)(N,N-diallylcarbonylmethyl)ammonium cation, and the like.

The organic quaternary ammonium cations having two or more ethylenically unsaturated double bonds are, for example, the following organic quaternary ammonium cations.

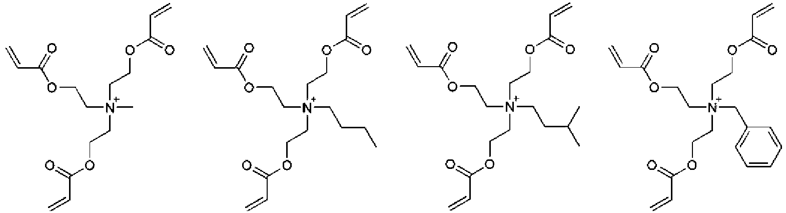

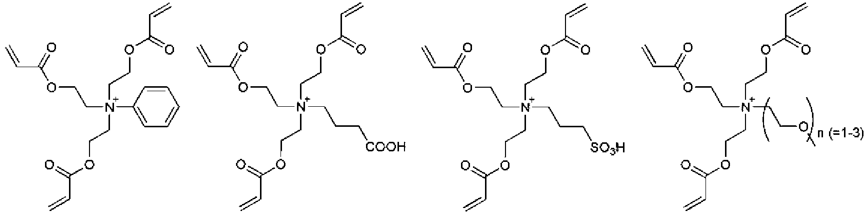

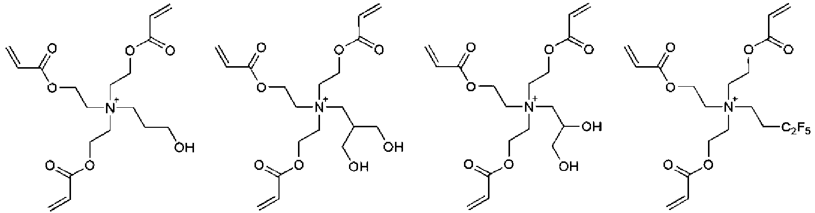

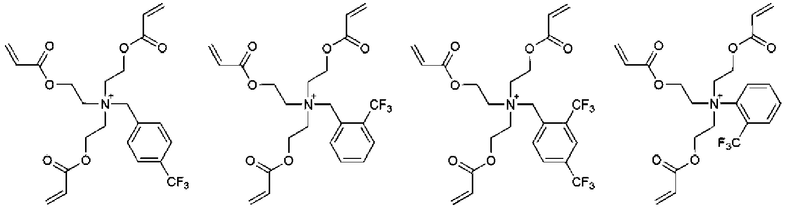

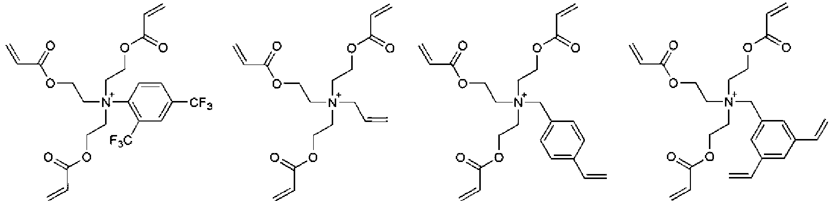

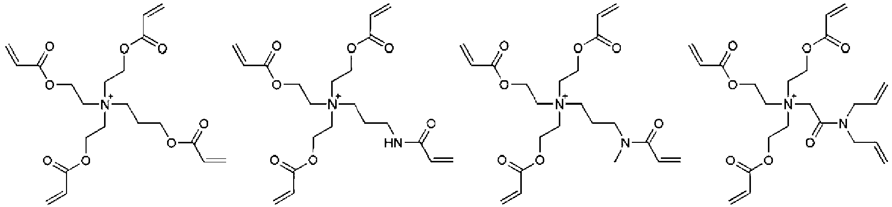

When R^{1A} and R^{1B} in the organic quaternary ammonium cation represented by the general formula (II) have one or more styryl group, and when D^{2A} and D^{2B} in the organic quaternary ammonium cation represented by the general formula (III) have one or more styryl group, specific examples include, for example, but are not limited to, a methyl(tris(2-(4-vinylbenzoyloxy)ethyl))ammonium cation, a n-butyl(tris(2-(4-vinylbenzoyloxy)ethyl))ammonium cation, an isopentyl(tris(2-(4-vinylbenzoyloxy)ethyl))ammonium cation, a phenyl(tris(2-(4-vinylbenzoyloxy)ethyl))ammonium cation, a benzyl(tris(2-(4-vinylbenzoyloxy)ethyl))ammonium cation, a 3-sulfopropyl(tris(2-(4-vinylbenzoyloxy)ethyl))ammonium cation, a 3-carboxypropyl(tris(2-(4-vinylbenzoyloxy)ethyl))ammonium cation, a 3-methoxyethyl(tris(2-(4-vinylbenzoyloxy)ethyl))ammonium cation,
a 3-hydroxypropyl(tris(2-(4-vinylbenzoyloxy)ethyl))ammonium cation, a (3-hydroxy(2-hydroxymethyl)propyl)(tris(2-(4-vinylbenzoyloxy)ethyl))ammonium cation, a 2,3-dihydropropyl(tris(2-(4-vinylbenzoyloxy)ethyl))ammonium cation, a 2-trifluoromethylphenyl(tris(2-(4-vinylbenzoyloxy)ethyl))ammonium cation, a 4-trifluoromethylphenyl(tris(2-(4-vinylbenzoyloxy)ethyl))ammonium cation, a 2-trifluoromethylbenzyl(tris(2-(4-vinylbenzoyloxy)ethyl))ammonium cation, a (2,5-bis(trifluoromethyl)benzyl)(tris(2-(4-vinylbenzoyloxy)ethyl))ammonium cation, a 4,4,4-trifluorobutyl(tris(2-(4-vinylbenzoyloxy)ethyl))ammonium cation,
a 4,4,5,5,5-pentafluopentyl(tris(2-(4-vinylbenzoyloxy)ethyl))ammonium cation, an allyl(tris(2-(4-vinylbenzoyloxy)ethyl))ammonium cation, a 4-vinylbenzyl(tris(2-(4-vinylbenzoyloxy)ethyl))ammonium cation, a 3-acryloyloxypropyl(tris(2-(4-vinylbenzoyloxy)ethyl))ammonium cation, a 3-N-methylacrylamidopropyl(tris(2-(4-vinylbenzoyloxy)ethyl))ammonium cation, and the like.

The organic quaternary ammonium cations having two or more ethylenically unsaturated double bonds are, for example, the following organic quaternary ammonium cations.

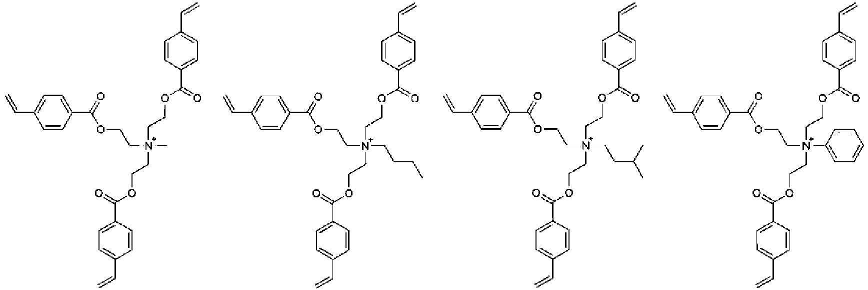

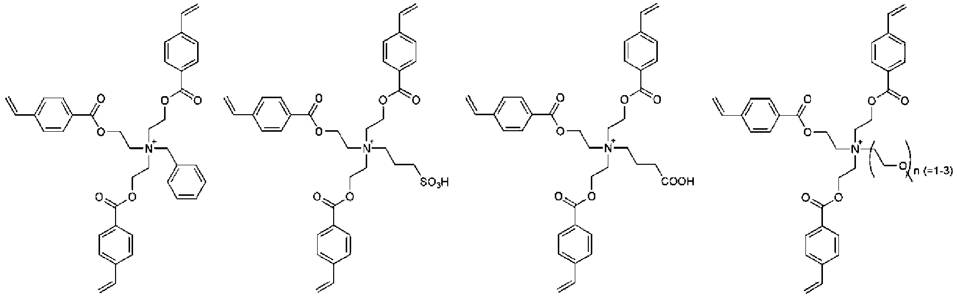

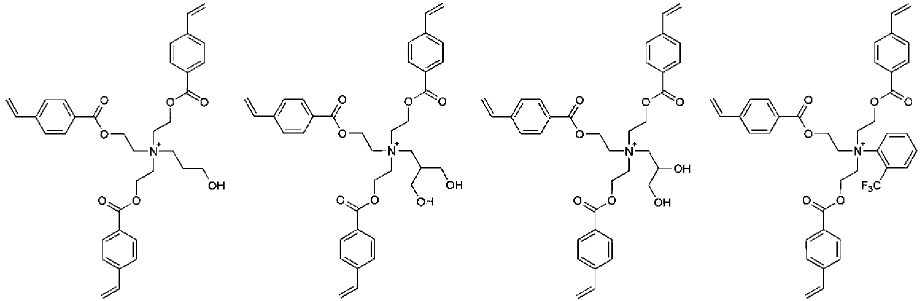

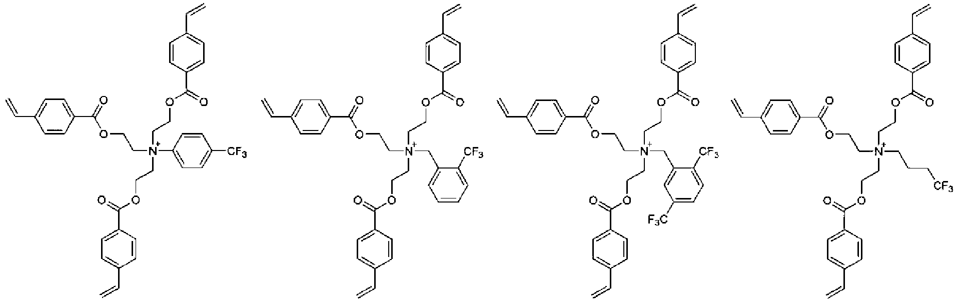

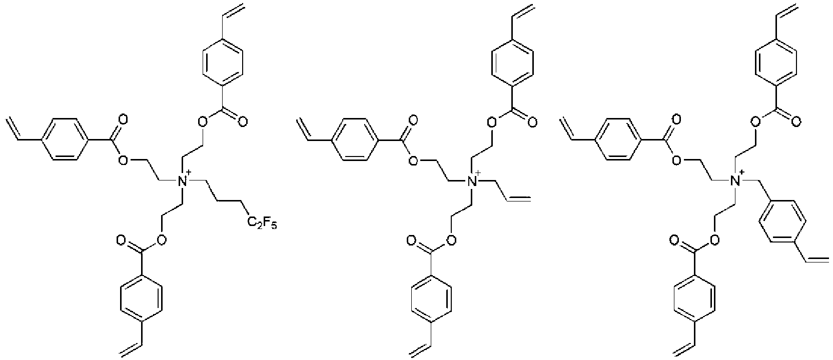

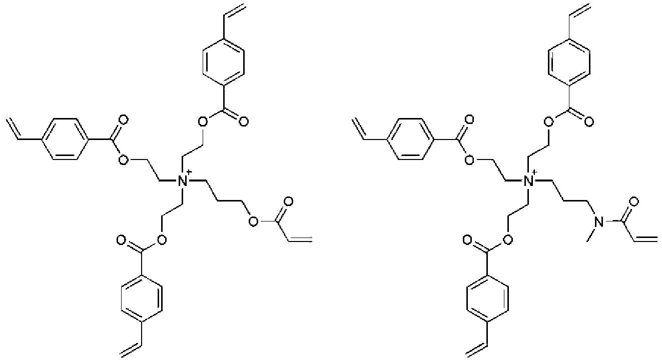

### [3-1-2. Organic Quaternary Ammonium Dication]

The organic quaternary ammonium dication having two or more ethylenically unsaturated double bonds according to the present embodiment is not particularly limited and may be a known and commonly used organic quaternary ammonium dication. The ethylenically unsaturated double bond may be derived from, for example, a vinyl group, a (meth)acryl group, a (meth)acrylamide group, or a styryl group.

The organic quaternary ammonium dication having two or more ethylenically unsaturated double bonds according to the present embodiment is, for example, an organic quaternary ammonium dication represented by the following general formula (IV). 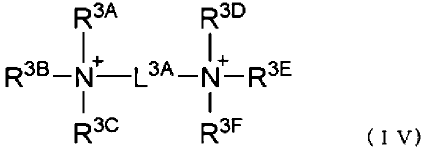 [wherein
R^{3A} to R^{3F} each independently denote a C₁₋₁₈ hydrocarbyl group, a 3- to 18-membered non-aromatic heterocyclic group, or a 5- to 18-membered aromatic heterocyclic group, the hydrocarbyl group and the heterocyclic groups each optionally having a substituent,
a divalent carbon atom at any position of R^{3A} to R^{3F} except the terminal positions may be substituted with -O-, -C(=O)-, -C(=O)O-, -OCO-, -CONH-, -NHCO-, -NH(C=O)O-, -S-, or -SO₂- (provided that adjacent divalent carbon atoms are not substituted at the same time),
L^{3A} denotes an optionally substituted C₁₋₁₈ hydrocarbylene group,
a divalent carbon atom at any position of L^{3A} may be substituted with -O-, -C(=O)-, -C(=O)O-, -OCO-, -CONH-, -NHCO-, -S-, or SO₂- (provided that adjacent divalent carbon atoms are not substituted at the same time),
<1> two or more hydrogen atoms in R^{3A} are substituted with (l) a vinyl group, (m) an allyl group, (n) a (meth)acryl group, (o) a (meth)acrylamide group, or (p) a styryl group, and
a hydrogen atom other than the above in R^{3A} and a hydrogen atom in R^{3B} to R^{3F} and L^{3A} may be substituted with (a) a fluorine atom, (b) a chlorine atom, (c) a bromine atom, (d) a C₁₋₃ haloalkyl group, (e) a hydroxy group, (f) a thiol group, (g) a nitro group, (h) a cyano group, (i) a carboxy group, (j) an amino group, (k) a sulfo group, (l) a vinyl group, (m) an allyl group, (n) a (meth)acryl group, (o) a (meth)acrylamide group, (p) a styryl group, (q) an epoxy group, (r) a glycidyl group, or (s) a group in which a hydrogen atom of a carboxy group or a hydroxy group is substituted with an acid-dissociable group,
<2> one or more hydrogen atoms in R^{3A} and R^{3B} is substituted with (l) a vinyl group, (m) an allyl group, (n) a (meth)acryl group, (o) a (meth)acrylamide group, or (p) a styryl group, and
a hydrogen atom other than the above in R^{3A} and R^{3B} and a hydrogen atom in R^{3C} to R^{3F} and L^{3A} may be substituted with (a) a fluorine atom, (b) a chlorine atom, (c) a bromine atom, (d) a C₁₋₃ haloalkyl group, (e) a hydroxy group, (f) a thiol group, (g) a nitro group, (h) a cyano group, (i) a carboxy group, (j) an amino group, (k) a sulfo group, (l) a vinyl group, (m) an allyl group, (n) a (meth)acryl group, (o) a (meth)acrylamide group, (p) a styryl group, (q) an epoxy group, (r) a glycidyl group, or (s) a group in which a hydrogen atom of a carboxy group or a hydroxy group is substituted with an acid-dissociable group, or
<3> one or more hydrogen atom in R^{3A} and R^{3D} is substituted with (l) a vinyl group, (m) an allyl group, (n) a (meth)acryl group, (o) a (meth)acrylamide group, or (p) a styryl group, and
a hydrogen atom other than the above in R^{3A} and R^{3D} and a hydrogen atom in a hydrogen atom in R^{3B}, R^{3C}, R^{3E}, R^{3F}, and L^{3A} may be substituted with (a) a fluorine atom, (b) a chlorine atom, (c) a bromine atom, (d) a C₁₋₃ haloalkyl group, (e) a hydroxy group, (f) a thiol group, (g) a nitro group, (h) a cyano group, (i) a carboxy group, (j) an amino group, (k) a sulfo group, (l) a vinyl group, (m) an allyl group, (n) a (meth)acryl group, (o) a (meth)acrylamide group, (p) a styryl group, (q) an epoxy group, (r) a glycidyl group, or (s) a group in which a hydrogen atom of a carboxy group or a hydroxy group is substituted with an acid-dissociable group, and}
R^{3B} and R^{3E}, and/or R^{3C} and R^{3F} may be directly bonded to each other by a single bond or may be bonded to each other via a divalent linking group -O-, -S-, -C(=O)-, -S(=O)-, -S(=O)₂-, -C(=O)O-, or a C₁₋₃ alkylene group to form a ring together with the nitrogen atom in the formula (IV)]

Specific examples of the organic quaternary ammonium dication represented by the general formula (IV) include, but are not limited to, a 1,4-diallyl-1,4-diazabicyclo[2.2.2]octane-1,4-diium, a 1,4-di(2-(meth)acryloyloxyethyl)-1,4-diazabicyclo[2.2.2]octane-1,4-diium, 1,4-bis(2-(meth)acrylamidoethyl)-1,4-diazabicyclo[2.2.2]octane-1,4-diium, and the like.

### [3-1-3. Pyridinium Cation]

Another pyridinium cation having two or more ethylenically unsaturated double bonds according to the present embodiment is, for example, a pyridinium cation represented by the following general formula (V). 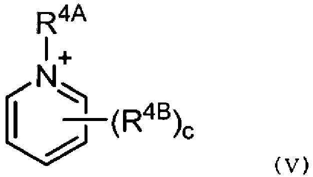 [wherein
R^{4A} denotes a C₁₋₁₈ hydrocarbyl group, a 3- to 18-membered non-aromatic heterocyclic group, or a 5- to 18-membered aromatic heterocyclic group, the hydrocarbyl group and the heterocyclic groups each optionally having a substituent,
a divalent carbon atom at any position of R^{4A} except the terminal positions and a binding portion thereof may be substituted with -O-, -C(=O)-, -C(=O)O-, -OCO-, -CONH-, -NHCO-, -NH(C=O)O-, -S-, or -SO2- (provided that adjacent divalent carbon atoms are not substituted at the same time),
a hydrogen atom in R^{4A} may be substituted with (a) a fluorine atom, (b) a chlorine atom, (c) a bromine atom, (d) a haloalkyl group, (e) a hydroxy group, (f) a thiol group, (g) a nitro group, (h) a cyano group, (i) a carboxy group, (j) an amino group, (k) a sulfo group, (l) a vinyl group, (m) an allyl group, (n) a (meth)acryloyl group, (o) a (meth)acryloyloxy group, (p) a (meth)acrylamide group, (q) a styryl group, (r) an epoxy group, (s) a glycidyl group, (t) an amide group, (u) a hydroxy group or a carboxy group each having a protective group, or (v) a C₁₋₁₈ hydrocarbyl group, a C₁₋₁₈ hydrocarbyloxy group, a C₁₋₁₈ hydrocarbylcarbonyl group, a C₁₋₁₈ hydrocarbylcarbonyloxy group, a C₁₋₁₈ hydrocarbyloxycarbonyl group, a C₁₋₁₈ hydrocarbyloxycarbonyloxy group, a C₁₋₁₈ hydrocarbylamino group, a di-C₁₋₁₈ hydrocarbylamino group, a C₁₋₁₈ hydrocarbylaminocarbonyl group, a di-C₁₋₁₈ hydrocarbylaminocarbonyl group, a C₁₋₁₈ hydrocarbylcarbonylamino group, a C₁₋₁₈ hydrocarbylaminocarbonyloxy group, a di-C₁₋₁₈ hydrocarbylaminocarbonyloxy group, a C₁₋₁₈ hydrocarbylaminocarbonylamino group, a di-C₁₋₁₈ hydrocarbylaminocarbonylamino group, a C₁₋₁₈ hydrocarbyloxycarbonylamino group, or a C₁₋₁₈ hydrocarbylthio group, in which at least part of hydrogen atoms may be substituted with (a) to (u), and a divalent carbon atom at any position of these substituents except the terminal positions may be substituted with -O-, -C(=O)-, -C(=O)O-, -OCO-, -CONH-, - NHCO-, -NH(C=O)O-, -S-, or -SO₂- (provided that adjacent divalent carbon atoms are not substituted at the same time),
R^{4B} each independently denotes a substituent, or a C₁₋₁₈ hydrocarbyl group, a 3- to 18-membered non-aromatic heterocyclic group, or a 5- to 18-membered aromatic heterocyclic group, the hydrocarbyl group and the heterocyclic groups each optionally having a substituent,
a divalent carbon atom at any position of R^{4B} except the terminal positions and a binding portion thereof may be substituted with -O-, -C(=O)-, -C(=O)O-, -OCO-, -CONH-, -NHCO-, -NH(C=O)O-, -S-, or -SO₂- (provided that adjacent divalent carbon atoms are not substituted at the same time),
a hydrogen atom in R^{4B} may be substituted with (a) a fluorine atom, (b) a chlorine atom, (c) a bromine atom, (d) a haloalkyl group, (e) a hydroxy group, (f) a thiol group, (g) a nitro group, (h) a cyano group, (i) a carboxy group, (j) an amino group, (k) a sulfo group, (l) a vinyl group, (m) an allyl group, (n) a (meth)acryloyl group, (o) a (meth)acryloyloxy group, (p) a (meth)acrylamide group, (q) a styryl group, (r) an epoxy group, (s) a glycidyl group, (t) an amide group, (u) a hydroxy group or a carboxy group each having a protective group, or (v) a C₁₋₁₈ hydrocarbyl group, a C₁₋₁₈ hydrocarbyloxy group, a C₁₋₁₈ hydrocarbylcarbonyl group, a C₁₋₁₈ hydrocarbylcarbonyloxy group, a C₁₋₁₈ hydrocarbyloxycarbonyl group, a C₁₋₁₈ hydrocarbyloxycarbonyloxy group, a C₁₋₁₈ hydrocarbylamino group, a di-C₁₋₁₈ hydrocarbylamino group, a C₁₋₁₈ hydrocarbylaminocarbonyl group, a di-C₁₋₁₈ hydrocarbylaminocarbonyl group, a C₁₋₁₈ hydrocarbylcarbonylamino group, a C₁₋₁₈ hydrocarbylaminocarbonyloxy group, a di-C₁₋₁₈ hydrocarbylaminocarbonyloxy group, a C₁₋₁₈ hydrocarbylaminocarbonylamino group, a di-C₁₋₁₈ hydrocarbylaminocarbonylamino group, a C₁₋₁₈ hydrocarbyloxycarbonylamino group, or a C₁₋₁₈ hydrocarbylthio group, in which at least part of hydrogen atoms may be substituted with (a) to (u), and a divalent carbon atom at any position of these substituents except the terminal positions may be substituted with -O-, -C(=O)-, -C(=O)O-, -OCO-, -CONH-, - NHCO-, -NH(C=O)O-, -S-, or -SO₂- (provided that adjacent divalent carbon atoms are not substituted at the same time),
c denotes an integer of 1 or more and 5 or less,
<1> two or more hydrogen atoms in R^{4A} is substituted with (l) a vinyl group, (m) an allyl group, (n) a (meth)acryl group, (o) a (meth)acrylamide group, or (p) a styryl group,
<2> one or more hydrogen atoms in R^{4A} are substituted with (l) a vinyl group, (m) an allyl group, (n) a (meth)acryl group, (o) a (meth)acrylamide group, or (p) a styryl group, and R^{4B} denotes (l) a vinyl group, (m) an allyl group, (n) a (meth)acryl group, (o) a (meth)acrylamide group, or (p) a styryl group, or one or more hydrogen atoms in R^{4B} are substituted with (l) a vinyl group, (m) an allyl group, (n) a (meth)acryl group, (o) a (meth)acrylamide group, or (p) a styryl group,
<3> c denotes an integer of 2 or more,
two or more R^{4B}s each independently denote (l) a vinyl group, (m) an allyl group, (n) a (meth)acryl group, (o) a (meth)acrylamide group, or (p) a styryl group,
two or more R^{4B}s each independently have one or more hydrogen atoms contained in R^{4B}s substituted with (l) a vinyl group, (m) an allyl group, (n) a (meth)acryl group, (o) a (meth)acrylamide group, or (p) a styryl group, or
one or more R^{4B}s each independently denote (l) a vinyl group, (m) an allyl group, (n) a (meth)acryl group, (o) a (meth)acrylamide group, or (p) a styryl group, and one or more R^{4B}s each independently have one or more hydrogen atoms contained in R^{4B}s substituted with (l) a vinyl group, (m) an allyl group, (n) a (meth)acryl group, (o) a (meth)acrylamide group, or (p) a styryl group, or
<4> two or more hydrogen atoms in R^{4B} are substituted with (l) a vinyl group, (m) an allyl group, (n) a (meth)acryl group, (o) a (meth)acrylamide group, or (p) a styryl group]

### [3-2. Another Counter Cation (A^{m+})]

Another counter cation (A^{m+}) according to the present embodiment is H⁺, a metal ion, or an ammonium ion.

A metal ion that can be used as "A^{m+}" is, for example, but not limited to, Li⁺, Na⁺, K⁺, Rb⁺, Cs⁺, Be²⁺, Mg²⁺, Ca²⁺, Sr²⁺, Ba²⁺, Al³⁺, Co³⁺, Bi³⁺, Zr⁴⁺, Hf⁴⁺, Bi⁵⁺, or the like. These metal ions may be used alone or in combination of two or more types thereof.

"A^{m+}" is preferably H⁺, Li⁺, Na⁺, K⁺, Rb⁺, Cs⁺, Mg²⁺, Ca²⁺, Sr²⁺, Ba²⁺, Al³⁺, Co³⁺, or NH⁴⁺, more preferably H⁺, Na⁺, K⁺, Rb⁺, Cs⁺, Ca²⁺, Al³⁺, Co³⁺, or NH⁴⁺.

### [3-3. Ratio of A^{m+} to Bⁿ⁺]

In the general formula (I), m denotes an integer in the range of 1 to 5, n denotes an integer of 1 or 2, a denotes a real number, and b denotes a real number greater than 0.

(A^{m+})ₐ(Bⁿ⁺)_{b}(C^{(am+bn)-}) (I)

Furthermore, a mixture of polyacid salts may contain a plurality of heteropolyacid salts having different ratios of A^{m+} to Bⁿ⁺. In this case, the values of a and b can be determined by, for example, NMR analysis, XRF analysis, XPS analysis, or the like.

The ratio of a/m to b/n depends on the types of the heteropolyoxoacid anion, A^{m+}, and Bⁿ⁺, and is preferably a/m:b/n = 0 to 3:3 to 12, more preferably 0 to 2:2 to 8, still more preferably 0 to 1:1 to 4.

In particular, when the value of a + b is a real number in the range of 3 to 7, and m and n are 1, it is preferable that a is a real number in the range of 0 to 3, and b is a real number in the range of 1 to 7, and it is more preferable that a is a real number in the range of 0 to 2, and b is a real number in the range of 2 to 7.

### EXAMPLES

The present invention will be more specifically described below with reference to examples, but the present invention is not limited to these examples.

### <Synthesis Example 1: Metatungstate Containing Five Diallyldimethylammonium Cations>

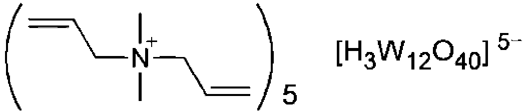

0.65 g (4.0 mmol, 6.0 eq) of diallyldimethylammonium chloride was dissolved in 20 mL of pure water to prepare "A". 2 g (0.67 mmol) of a commercially available ammonium metatungstate was dissolved in aqueous hydrochloric acid (pH = 1, 20 mL) to prepare "B". While "A" was stirred at room temperature, "B" was added thereto, and the resulting mixture was stirred for 4 hours. The resulting precipitate was suction-filtered and washed with pure water and methanol. The precipitate was then dried overnight under reduced pressure to produce 1.5 g of the target compound.
¹H-NMR (400 MHz, DMSO-d6): δ (ppm) = 3.01 (s, 30H), 4.02 (d, 20H), 5.62-5.68 (m, 20H), 6.04-6.14 (m, 10H), 6.71 (2, 3H).
¹⁸³W-NMR (20.84 MHz, DMSO-d6): δ (ppm) = -100.13 (s, 12W).
ESI-MS: POSITIVE m/z 126.13 ([C₈H₁₆N]⁺)
NEGATIVE m/z 721.3 (median) ([H₄W₁₂O₄₀]⁴⁻)

### <Synthesis Example 2: tris(tetraallylammonium)phosphotungstate>

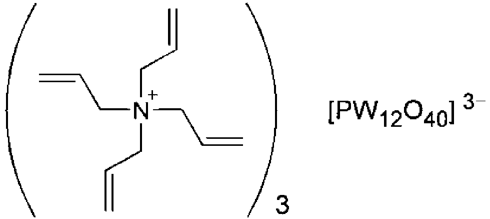

0.58 g (2.7 mmol, 4.0 eq) of tetraallylammonium iodide was dissolved in 20 mL of pure water to prepare "A". A commercially available phosphotungstic acid (2 g, 0.69 mmol) was then dissolved in 20 mL of pure water to prepare "B". While "A" was stirred at room temperature, "B" was added thereto, and the resulting mixture was stirred for 4 hours. The resulting precipitate was suction-filtered and washed with methanol. The precipitate was then dried overnight under reduced pressure to produce 1.7 g of the target compound.
¹H-NMR (400 MHz, DMSO-d6): δ (ppm) = 3.90 (d, 24H), 5.66-5.72 (m, 24H), 6.05-6.14 (m, 12H).
¹⁸³W-NMR (20.84 MHz, DMSO-d6): δ (ppm) = -86.7 (s, 12W).
ESI-MS: POSITIVE m/z 178.16 ([C₁₂H₂₀N]⁺)
NEGATIVE m/z 959 (median) ([PW₁₂O₄₀]³⁻)

### <Synthesis Example 3: tetrakis(tetraallylammonium)silicotungstate>

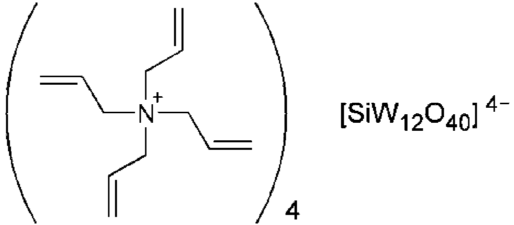

1.0 g (3.5 mmol, 5.0 eq) of tetraallylammonium iodide was dissolved in 20 mL of pure water to prepare "A". 2 g (0.7 mmol) of a commercially available silicotungstic acid was dissolved in 20 mL of pure water to prepare "B". While "A" was stirred at room temperature, "B" was added thereto, and the resulting mixture was stirred for 4 hours. The resulting precipitate was suction-filtered and washed with methanol. The precipitate was then dried overnight under reduced pressure to produce 2.1 g of the target compound.
¹H-NMR (400 MHz, DMSO-d6): δ (ppm) = 3.90 (d, 32H), 5.66-5.72 (m, 32H), 6.05-6.14 (m, 16H).
¹⁸³W-NMR (20.84 MHz, DMSO-d6): δ (ppm) = -92.7 (s, 12W).
ESI-MS: POSITIVE m/z 178.16 ([C₁₂H₂₀N]⁺)
NEGATIVE m/z 718.5 (median) ([SiW₁₂O₄₀]⁴⁻)

### <Synthesis Example 4: Metatungstate Containing Five Tetraallylammonium Cations>

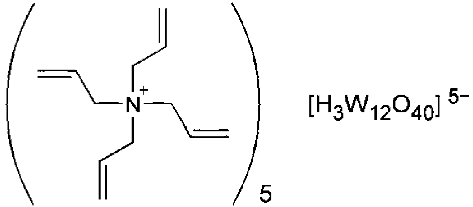

1.2 g (4.1 mmol, 6.0 eq) of tetraallylammonium iodide was dissolved in 20 mL of pure water to prepare "A". 2 g (0.67 mmol) of a commercially available ammonium metatungstate was then dissolved in aqueous hydrochloric acid (pH = 1, 20 mL) to prepare "B". While "A" was stirred at room temperature, "B" was added thereto, and the resulting mixture was stirred for 4 hours. The resulting precipitate was suction-filtered and washed with pure water and methanol. The precipitate was then dried overnight under reduced pressure to produce 2.2 g of the target compound.
¹H-NMR (400 MHz, DMSO-d6): δ (ppm) = 3.90 (d, 40H), 5.66-5.72 (m, 40H), 6.05-6.14 (m, 20H), 6.71 (s, 3H).
¹⁸³W-NMR (20.84 MHz, DMSO-d6): δ (ppm) = -100.13 (s, 12W).
ESI-MS: POSITIVE m/z 178.16 ([C₁₂H₂₀N]⁺)
NEGATIVE m/z 721.3 (median) ([H₄W₁₂O₄₀]⁴⁻)

### <Synthesis Example 5: Metatungstate Salt Containing Five Triallyl-4-vinylbenzylammonium Cations>

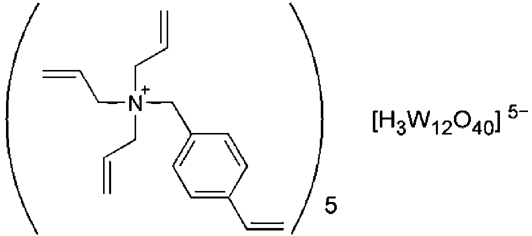

1.2 g (4.1 mmol, 6.0 eq) of triallyl-4-vinylbenzylammonium chloride was dissolved in 20 mL of pure water to prepare "A". 2 g (0.67 mmol) of a commercially available ammonium metatungstate was then dissolved in aqueous hydrochloric acid (pH = 1, 20 mL) to prepare "B". While "A" was stirred at room temperature, "B" was added thereto, and the resulting mixture was stirred for 4 hours. The resulting precipitate was suction-filtered and washed with pure water and methanol. The precipitate was then dried overnight under reduced pressure to produce 2.4 g of the target compound.
¹H-NMR (400 MHz, DMSO-d6): δ (ppm) = 3.33 (t, 30H), 4.57 (s, 10H), 5.35-5.42 (m, 5H), 5.65-5.75 (m, 30H), 5.90-6.0 (d, 5H), 6.10-6.30 (m, 15H), 6.7-6.9 (m, 8H), 7.61 (s, 20H). ¹⁸³W-NMR (20.84 MHz, DMSO-d6): δ (ppm) = -100.13 (s, 12W).
ESI-MS: POSITIVE m/z 254.19 ([C₁₈H₂₄N]⁺)
NEGATIVE m/z 721.3 (median) ([H₄W₁₂O₄₀]⁴⁻)

### <Synthesis Example 6: tris(triallyl(2-trifluoromethylbenzyl)ammonium)phosphotungstate>

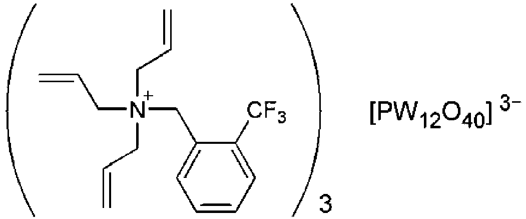

1.0 g (2.8 mmol, 4.0 eq) of triallyl(2-trifluoromethylbenzyl)ammonium bromide was dissolved in 20 mL of pure water to prepare "A". 2 g (0.69 mmol) of a commercially available phosphotungstic acid was dissolved in 20 mL of pure water to prepare "B". While "A" was stirred at room temperature, "B" was added thereto, and the resulting mixture was stirred for 4 hours. The resulting precipitate was suction-filtered and washed with methanol. The precipitate was then dried overnight under reduced pressure to produce 2.5 g of the target compound.
¹H-NMR (400 MHz, DMSO-d6): δ (ppm) = 4.02 (d, 18H), 4.71 (m, 6H), 5.60-5.80 (m, 18H), 5.90-6.0 (d, 15H), 5.98-6.25 (m, 9H), 7.75-7.98 (m, 12H).
¹⁸³W-NMR (20.84 MHz, DMSO-d6): δ (ppm) = -86.7 (s, 12W).
ESI-MS: POSITIVE m/z 296.16 ([C₁₇H₂₁F₃N]⁺)
NEGATIVE m/z 959 (median) ([PW₁₂O₄₀]³⁻)

### <Synthesis Example 7: tetrakis(triallyl(2-trifluoromethylbenzyl)ammonium)silicotungstate>

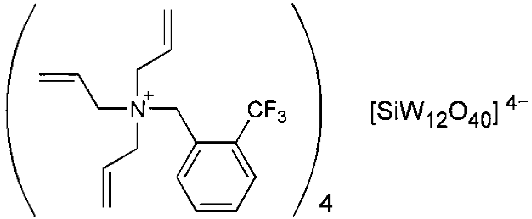

1.3 g (3.5 mmol, 5.0 eq) of triallyl(2-trifluoromethylbenzyl)ammonium bromide was dissolved in 20 mL of pure water to prepare "A". 2 g (0.7 mmol) of a commercially available silicotungstic acid was then dissolved in 20 mL of pure water to prepare "B". While "A" was stirred at room temperature, "B" was added thereto, and the resulting mixture was stirred for 4 hours. The resulting precipitate was suction-filtered and washed with methanol. The precipitate was then dried overnight under reduced pressure to produce 1.9 g of the target compound.
¹H-NMR (400 MHz, DMSO-d6): δ (ppm) = 4.02 (d, 24H), 4.71 (s, 8H), 5.60-5.8 (m, 24H), 5.90-6.0 (d, 20H), 5.98-6.25 (m, 12H), 7.75-7.98 (m, 16H).
¹⁸³W-NMR (20.84 MHz, DMSO-d6): δ (ppm) = -92.7 (s, 12W).
ESI-MS: POSITIVE m/z 296.16 ([C₁₇H₂₁F₃N]⁺)
NEGATIVE m/z 718.5 (median) ([SiW₁₂O₄₀]⁴⁻)

### <Synthesis Example 8: Metatungstate Salt Containing Five Triallyl(2-trifluoromethylbenzyl)ammonium Cations>

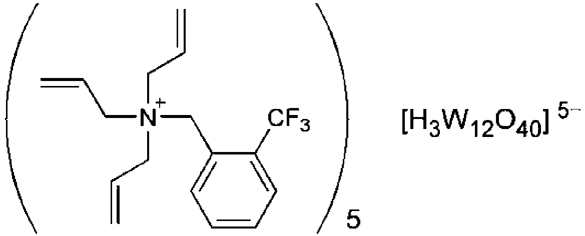

1.6 g (4.1 mmol, 6.0 eq) of triallyl(2-trifluoromethylbenzyl)ammonium bromide was dissolved in 20 mL of pure water to prepare "A". 2 g (0.67 mmol) of a commercially available ammonium metatungstate was then dissolved in aqueous hydrochloric acid (pH = 1, 20 mL) to prepare "B". While "A" was stirred at room temperature, "B" was added thereto, and the resulting mixture was stirred for 4 hours. The resulting precipitate was suction-filtered and washed with pure water and methanol. The precipitate was then dried overnight under reduced pressure to produce 2.3 g of the target compound.
¹H-NMR (400 MHz, DMSO-d6): δ (ppm) = 4.02 (d, 30H), 4.73 (s, 10H), 5.60-5.8 (m, 30H), 5.98-6.25 (m, 15H), 6.71 (s, 3H), 7.75-7.98 (m, 20H).
¹⁸³W-NMR (20.84 MHz, DMSO-d6): δ (ppm) = -100.13 (s, 12W).
ESI-MS: POSITIVE m/z 296.16 ([C₁₇H₂₁F₃N]⁺)
NEGATIVE m/z 721.3 (median) ([H₄W₁₂O₄₀]⁴⁻)

### <Synthesis Example 9: Metatungstate Salt Containing Five Triallyl(3-hydroxypropyl)ammonium Cations>

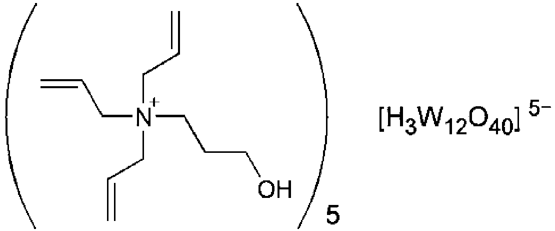

1.34 g (4.1 mmol, 6.0 eq) of triallyl(3-hydroxypropyl)ammonium bromide and 2.0 g (0.67 mmol) of ammonium metatungstate were dissolved in 20 mL of pure water and were stirred for 30 minutes. After water was removed by evaporator distillation, 2 mL of pure water was further added. The resulting powder was suction-filtered and dried under reduced pressure to produce 0.3 g of the target compound.
¹H-NMR (400 MHz, DMSO-d6): δ (ppm) = 1.85-2.0 (m, 10H), 2.20-2.30 (m, 10H), 3.9-4.0 (m, 30H), 4.5-5.0 (br, 5H), 5.6-5.8 (m, 30H), 6.0-6.2 (m, 15H), 6.71 (s, 3H).
¹⁸³W-NMR (20.84 MHz, DMSO-d6): δ (ppm) = -100.13 (s, 12W).
ESI-MS: POSITIVE m/z 196.17 ([C₁₂H₂₂NO]⁺)
NEGATIVE m/z 721.3 (median) ([H₄W₁₂O₄₀]⁴⁻)

### <Synthesis Example 10: tris(dimethyldi(2-(N-methylacrylamido)ethyl)ammonium)phosphotungstate salt>

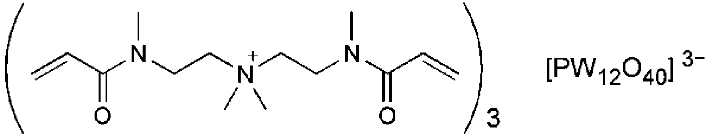

1.16 g (2.8 mmol, 4.0 eq) of dimethyldi(2-(N-methylacrylamido)ethyl)ammonium triflate was dissolved in 20 mL of pure water to prepare "A". 2 g (0.69 mmol) of a commercially available phosphotungstic acid was dissolved in 20 mL of pure water to prepare "B". While "A" was stirred at room temperature, "B" was added thereto, and the resulting mixture was stirred for 4 hours. The resulting precipitate was suction-filtered and washed with methanol. The precipitate was then dried overnight under reduced pressure to produce of 1.2 g of the target compound.
¹H-NMR (400 MHz, DMSO-d6): δ (ppm) = 2.90-3.2 (m, 36H), 3.45-3.6 (m, 12H), 3.75-3.95 (m, 12H), 5.75 (d, 6H), 6.18 (d, 6H), 6.7-6.9 (m, 6H).
¹⁸³W-NMR (20.84 MHz, DMSO-d6): δ (ppm) = -86.7 (s, 12W).
ESI-MS: POSITIVE m/z 268.20 ([C₁₄H₂₆N₃O₂]⁺)
NEGATIVE m/z 959 (median) ([PW₁₂O₄₀]³⁻)

### <Synthesis Example 11: tetrakis(dimethyldi(2-(N-methylacrylamido)ethyl)ammonium)silicotungstate salt>

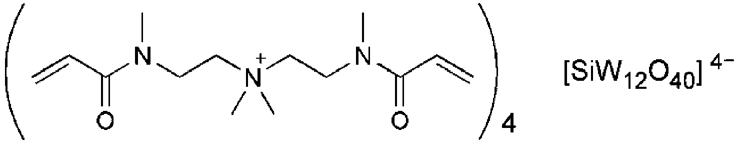

1.45 g (3.5 mmol, 5.0 eq) of dimethyldi(2-(N-methylacrylamido)ethyl)ammonium triflate was dissolved in 20 mL of pure water to prepare "A". 2 g (0.7 mmol) of a commercially available silicotungstic acid was dissolved in 20 mL of pure water to prepare "B". While "A" was stirred at room temperature, "B" was added thereto, and the resulting mixture was stirred for 4 hours. The resulting precipitate was suction-filtered and washed with methanol. The precipitate was then dried overnight under reduced pressure to produce of 1.4 g of the target compound.
¹H-NMR (400 MHz, DMSO-d6): δ (ppm) = 2.90-3.2 (m, 48H), 3.45-3.6 (m, 16H), 3.75-3.95 (m, 16H), 5.75 (d, 8H), 6.18 (d, 8H), 6.7-6.9 (m, 8H).
¹⁸³W-NMR (20.84 MHz, DMSO-d6): δ (ppm) = -92.7 (s, 12W).
ESI-MS: POSITIVE m/z 268.20 ([C₁₄H₂₆N₃O₂]⁺)
NEGATIVE m/z 718.5 (median) ([SiW₁₂O₄₀]³⁻)

### <Synthesis Example 12: Metatungstate Salt Containing Five Dimethyldi(2-(N-Methylacrylamido)ethyl)ammonium Cations>

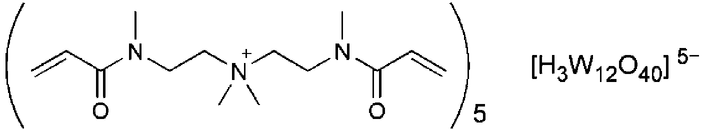

1.7 g (4.1 mmol, 6.0 eq) of dimethyldi(2-(N-methylacrylamido)ethyl)ammonium triflate was dissolved in 20 mL of pure water to prepare "A". 2 g (0.67 mmol) of a commercially available ammonium metatungstate was dissolved in aqueous hydrochloric acid (pH = 1, 20 mL) to prepare "B". While "A" was stirred at room temperature, "B" was added thereto, and the resulting mixture was stirred for 4 hours. The resulting precipitate was suction-filtered and washed with pure water and methanol. The precipitate was then dried overnight under reduced pressure to produce 2.3 g of the target compound.
¹H-NMR (400 MHz, DMSO-d6): δ (ppm) = 2.90-3.2 (m, 60H), 3.45-3.6 (m, 20H), 3.75-3.95 (m, 20H), 5.75 (d, 10H), 6.18 (d, 10H), 6.7-6.9 (m, 13H).
¹⁸³W-NMR (20.84 MHz, DMSO-d6): δ (ppm) = -100.13 (s, 12W).
ESI-MS: POSITIVE m/z 268.20 ([C₁₄H₂₆N₃O₂]⁺)
NEGATIVE m/z 721.3 (median) ([H₄W₁₂O₄₀]⁴⁻)

## Claims

1. A polyacid salt represented by the general formula (I) or a mixture thereof.
(A^{m+})ₐ(Bⁿ⁺)_{b}(C^{(am+bn)-}) (I):
wherein
A^{m+} each independently denotes H⁺, a metal ion, or an ammonium ion,
Bⁿ⁺ each independently denotes an organic quaternary ammonium cation, an organic quaternary ammonium dication, or a pyridinium cation, each having two or more ethylenically unsaturated double bonds,
C^{(am+bn)-} denotes a polyacid anion, and
m denotes an integer in the range of 1 to 5, n denotes an integer of 1 or 2, a denotes a real number, and b denotes a real number greater than 0.

2. The polyacid salt or a mixture thereof according to claim 1, wherein the polyacid anion is an isopolyoxomolybdate anion, an isopolyoxotungstate anion, an isopolyoxoniobate anion, or an isopolyoxotantalate anion.

3. The polyacid salt or a mixture thereof according to claim 1, wherein the polyacid anion is a heteropolyacid anion, and the heteropolyacid anion includes Mo, W, V, Nb, or Ta as a polyatom and includes P, Si, B, S, or Ge as a heteroatom.

4. The polyacid salt or a mixture thereof according to claim 1, wherein the ethylenically unsaturated double bond is derived from a vinyl group, a (meth)acryloyl group, a (meth)acryloyloxy group, a (meth)acrylamide group, or a styryl group.

5. The polyacid salt or a mixture thereof according to claim 1, wherein the organic quaternary ammonium cation having two or more ethylenically unsaturated double bonds is an organic quaternary ammonium cation represented by the general formula (II): 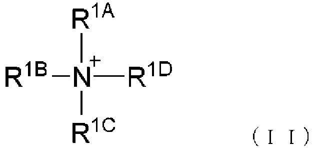 wherein
R^{1A} to R^{1D} each independently denote a C₁₋₁₈ hydrocarbyl group, a 3- to 18-membered non-aromatic heterocyclic group, or a 5- to 18-membered aromatic heterocyclic group, the hydrocarbyl group and the heterocyclic groups each optionally having a substituent,
a divalent carbon atom at any position of R^{1A} to R^{1D} except terminal positions may be substituted with -O-, -C(=O)-, -C(=O)O-, -OCO-, -CONH-, -NHCO-, -NH(C=O)O-, -S-, or -SO₂- (provided that adjacent divalent carbon atoms are not substituted at the same time),
a hydrogen atom in R^{1A} to R^{1D} may be substituted with (a) a fluorine atom, (b) a chlorine atom, (c) a bromine atom, (d) a haloalkyl group, (e) a hydroxy group, (f) a thiol group, (g) a nitro group, (h) a cyano group, (i) a carboxy group, (j) an amino group, (k) a sulfo group, (l) a vinyl group, (m) an allyl group, (n) a (meth)acryloyl group, (o) a (meth)acryloyloxy group, (p) a (meth)acrylamide group, (q) a styryl group, (r) an epoxy group, (s) a glycidyl group, (t) an amide group, (u) a hydroxy group or a carboxy group each having a protective group, or (v) a C₁₋₁₈ hydrocarbyl group, a C₁₋₁₈ hydrocarbyloxy group, a C₁₋₁₈ hydrocarbylcarbonyl group, a C₁₋₁₈ hydrocarbylcarbonyloxy group, a C₁₋₁₈ hydrocarbyloxycarbonyl group, a C₁₋₁₈ hydrocarbyloxycarbonyloxy group, a C₁₋₁₈ hydrocarbylamino group, a di-C₁₋₁₈ hydrocarbylamino group, a C₁₋₁₈ hydrocarbylaminocarbonyl group, a di-C₁₋₁₈ hydrocarbylaminocarbonyl group, a C₁₋₁₈ hydrocarbylcarbonylamino group, a C₁₋₁₈ hydrocarbylaminocarbonyloxy group, a di-C₁₋₁₈ hydrocarbylaminocarbonyloxy group, a C₁₋₁₈ hydrocarbylaminocarbonylamino group, a di-C₁₋₁₈ hydrocarbylaminocarbonylamino group, a C₁₋₁₈ hydrocarbyloxycarbonylamino group, or a C₁₋₁₈ hydrocarbylthio group, in which at least part of hydrogen atoms may be substituted with (a) to (u), and a divalent carbon atom at any position of these substituents except terminal positions may be substituted with -O-, -C(=O)-, -C(=O)O-, -OCO-, -CONH-, - NHCO-, -NH(C=O)O-, -S-, or -SO₂- (provided that adjacent divalent carbon atoms are not substituted at the same time),
at least one hydrogen atom in R^{1A} and R^{1B} is substituted with (l) a vinyl group, (m) an allyl group, (n) a (meth)acryloyl group, (o) a (meth)acryloyloxy group, (p) a (meth)acrylamide group, or (q) a styryl group, and
any two of R^{1A} to R^{1D} may be directly bonded to each other by a single bond or may be bonded to each other via a divalent linking group -S-, -C(=O)-, -S(=O)-, -S(=O)₂-, - C(=O)O-, -C(=O)NH-, or a C₁₋₃ alkylene group to form a ring together with a nitrogen atom in the formula (II).
